BAD ORIGINAL

**Europäisches Patentamt**

(19) **European Patent Office**

**Office européen des brevets**

(11) Publication number: **0 004 170**
**A1**

(12) # EUROPEAN PATENT APPLICATION

(21) Application number: **79300323.7**

(22) Date of filing: **05.03.79**

(51) Int. Cl.²: **A 24 C 1/04**
**G 06 F 15/20, B 26 D 5/34**

(30) Priority: 09.03.78 US 884849
02.05.78 US 902247
02.05.78 US 902253
02.05.78 US 902262
02.05.78 US 902263
02.05.78 US 902264
02.05.78 US 902245

(43) Date of publication of application:
19.09.79 Bulletin 79/19

(84) Designated contracting states:
BE CH DE FR GB IT LU NL SE

(71) Applicant: GULF & WESTERN CORPORATION
1 Gulf & Western Plaza
New York New York 10023 (US)

(72) Inventor: Sinclair, Robert I.
Roxciticus Road R.D. No. 2
Mendham New Jersey 07945 (US)

(72) Inventor: Anderson, Verner
R.D. No. 3-3103
Berwick Pennsylvania 18603 (US)

(72) Inventor: Locan, David J.
125 Karen Lee Road
Glastonbury Connecticut 06133 (US)

(72) Inventor: Rich, Leonard G.
7 Rye Ridge Parkway
West Hartford Connecticut 06117 (US)

(72) Inventor: Wood, Kenneth O.
27 Ludwig Road
Ellington Tolland Connecticut 06029 (US)

(72) Inventor: Hevenor, Charles M., Jr.
24 Toomey Lane
Bolton Hartford Connecticut 06040 (US)

(72) Inventor: Pavone, Robert J.
48 Norman Drive
South Windsor Connecticut 06074 (US)

(74) Representative: Abbie, Andrew Kenneth et al,
A.A. Thornton & Co 303-306 High Holborn
London WC1V 7LE (GB)

(54) Method, system and apparatus for cutting profiles from natural leaves.

(57) There is provided a method, system and apparatus for cutting a profile, such as a cigar wrapper, from a natural leaf, such as a tobacco leaf, wherein the leaf is spread and supported on a support surface in a random position (40). Thereafter, a leaf facsimile or image, including the leaf outline and surface imperfections, is developed (70) and at least one cut position for the wrapper is located in an area excluding the leaf outline and surface imperfections (80). This cut position is recorded and converted to digital data (100). The wrapper is then cut from the leaf at an actual cut position corresponding to the located cut position determined by the digital data (104). Also, the facsimile is created by a back lighting arrangement which illuminates color variations and surface imperfections, as well as the leaf outline, so that leaf colour variations can be included in the facsimile or image.

EP 0 004 170 A1

./...

FIG. 4

- 1 -

## Method, System and Apparatus for
## Cutting Profiles from Natural Leaves.

The present invention relates to the art of providing a flat profile having a desired shape from a natural leaf of the type having a general outline, a central stem, protruding and/or dark veins, surface color variations, surface imperfections, holes, and other light intensity detectable features. The profile is to be cut from a position that avoids the defect features of the natural leaf. More particularly the invention is directed toward a method, system and apparatus for cutting a cigar wrapper from a natural tobacco leaf.

An embodiment of the invention will be described with respect to the concept of cutting a cigar wrapper or wrappers from a natural tobacco leaf, which wrappers exclude the leaf edge and leaf defects, such as holes, even though the invention is envisioned as having more general application.

## BACKGROUND OF THE INVENTION

In the production of cigars, a core is usually provided. In practice, the core is formed either of small tobacco pieces or whole tobacco leaves bunched in a longitudinal direction. These cores are usually wrapped with a binder and then spirally wrapped by an elongated sheet, known as a "wrapper". The cigar wrapper is formed by cutting a given profile from a tobacco sheet product. In some instances, a wrapper is cut from a synthetic tobacco sheet. However, it is desirable to provide a natural tobacco leaf as the outer cigar wrapper to present an appealing appearance which facilitates marketing of the finished cigar. It is essential for a quality cigar that a natural tobacco leaf be used for the outer wrapper and that the finished cigar have no noticeable defect, such as a hole, a color variation, an edge portion of the leaf, or a stem. Indeed, certain unduly noticeable heavy veins should not be incorporated into a wrapper for a quality cigar. All of these concepts in wrapper selection are essential in the marketing of a cigar in the very competitive cigar industry. The wrapper is important in determining the overall visual concept that a purchaser forms regarding the quality of the cigar. Consequently, the wrapper must have an appearance that imparts an impression of quality to the resulting cigar.

As is well known in the cigar industry, the wrapper, which is helically disposed in overlapping fashion around the cigar, must also have a smooth outer appearance which is somewhat difficult to obtain since the shape of the cigar often varies along its length. For that reason, the wrapper must be cut in a complex shape and must be accurately spiraled around the cigar core and/or binder to produce the desired smooth outer appearance. This requirement, combined with the demand for a cigar wrapper with no surface defects, has made one of the more critical aspects of cigar making the system by which the cigar wrapper is cut from a natural tobacco leaf. This system is made quite complex by the fact that each natural tobacco leaf has different surface variations which must be excluded from a wrapper.

Due to the extreme criticality in producing quality cigar wrappers for use in cigar manufacturing, the wrappers are generally cut by a manual orienting procedure that has not changed substantially over the years. The process requires a skilled operator who manually orients a half of a leaf formed by removing the center stem. This leaf portion or half is examined for holes, coarse veins, or other visible imperfections on one surface. After this inspection, the leaf portion is usually spread onto a cutting surface including a cutting die surrounded by perforated surfaces through which vacuum can be applied to the spread leaf. The leaf is manually positioned over the cutting die to insure that the outline of the die, which has the shape of the cigar wrapper, does not include an edge portion or any other visible surface imperfection in the natural tobacco leaf. After this placement has been made, the vacuum is applied to hold the leaf in place on the cutting surface. A roller is forced over the leaf and cuts out a leaf portion determined by the profile of the cutter over which the leaf was positioned. After a first cut has been made, the vacuum is released, the wrapper is removed and the cut tobacco portion is again oriented by the operator for a second cut from the leaf half, if a second cut is possible without including any surface imperfection. The wrapper has an elongated shape and the leaf veins must have a predetermined diagonal orientation in the wrapper. Consequently, the general disposition of the wrapper cut must be generally parallel to the original stem of the natural tobacco leaf within a few degrees, such as $10°-15°$. In this manner, the veins, which are found on the leaf, will have the proper pattern when the wrapper is spirally wound around the core and/or binder to form a finished cigar. This process of manually orienting and then cutting is continued until no other wrapper can be cut from a leaf half. At this time, the leaf is removed from the cutting station for use in other tobacco products or discarded. Other procedures are used in the tobacco industry for producing the cigar wrappers. This particular description is representative in that each of the procedures involves manual manipulation of a leaf or a leaf half into a particular position wherein a cut is made in the natural tobacco leaf so as to avoid surface imperfections. In all instances, an operator, who must be skilled and highly trained, is required for the production of a quality wrapper produced from a natural tobacco leaf. In practice, the cost of producing the quality wrapper is a relatively high proportion of the cigar manufacturing costs in that the remainder of the cigar making process is generally mechanized and can be accomplished at relatively high processing speeds.

In view of this, there is a substantial demand for an arrangement wherein the cigar wrapper can be cut from a tobacco leaf in a high speed operation involving no manual manipulation without sacrificing the high quality required for the production of such wrappers. The advantage to the cigar manufacturing process of avoiding, or reducing, the manual manipulation required in producing the cigar wrappers is well known in the industry. 0004170

Before the leaves are stacked for use by an operator, the heavy stem or mid-rib of each tobacco leaf is removed. Each resulting half of the leaf is then "booked" in a separate pile for use in the cutting operation. Since the veins in the leaf extend diagonally in different general directions with respect to the stem, one half of the leaf is used for one cigar making machine and the other half of the leaf is used for another cigar making machine. This prevents mixing of wrappers from both leaf halves so that the diagonal veins within the cigar wrapper are uniform for each run of cigars. If whole leaves were provided to the operator for manual orientation and cutting, the cut wrappers could have different vein patterns unless the operator exercised extreme care and attention. Such mixing of vein patterns would not be acceptable in the production of cigars. The wrappers must be consistent in the orientation of the vein angles. Consequently, not only have prior arrangements for cutting cigar wrappers required manual manipulation, but they have required stemming of the tobacco leaf and grouping the leaves in "booked" and matched halves for use in a particular tobacco wrapping machine. Stemming, booking and other controls have increased the cost of wrappers without increasing their quality. All of these disadvantages are known in the cigar industry and attempts have been made to correct one or more of the various disadvantages experienced in the previously used arrangements for producing cigar wrappers from natural tobacco leaves.

The most common approach to solving the problems in cutting wrappers has been to mechanize or increase the speed of the cutting operation and the wrapper transfer operation. Such a concept is shown in United States Letters Patent 3,591,044. This patent is

- 6 -

0004170

incorporated herein by reference for background information only. Such an arrangement increases production by an operator, but it does not solve the basic problems involved in the efficient production of a wrapper from a natural tobacco leaf. Manual manipulation or orientation, stemming and booking of leaf halves are still required. This was the background situation presented when the present novel system was developed to cut cigar wrappers from natural tobacco leaves.

THE INVENTION

The present invention the scope of which is defined in the appended claims relates to a method, system and apparatus for automatically processing natural tobacco leaves and similar natural leaf products to produce a cut profile, such as a cigar wrapper, from the leaf while avoiding surface imperfections without requiring manual manipulation of the leaf and, as an added feature, without requiring removal of the stem from the leaf.

In accordance with an embodiment of the present invention there is provided a method of making a flat profile having a preselected shape, such as a cigar wrapper, from a natural leaf, such as a natural tobacco leaf. This type of leaf has surface variations and defects detectable by light rays. The method comprises the steps of supporting the leaf in a spread condition on a support surface at a random location, creating a leaf profile including the edge outline and surface imperfections of the leaf, locating at least one cut position within the outline and excluding the imperfections, and then cutting the profile from the leaf at a cut position corresponding to the located cut position. In this manner, a profile of the leaf itself is created and is used instead of the leaf itself, for identifying the cut positions. After the position or positions have been located, each wrapper is cut with a somewhat standard cutter concept at the position determined by the use of the constructed profile or facsimile or image. The natural leaf itself is used in the manual manipulation systems of the prior art for locating the cut positions of the cigar wrappers.

The system using a leaf facsimile to locate cut positions, in accordance with an aspect of the invention, develops cut coordinates in digital form for adjusting the leaf with respect to the wrapper cutter. These digital coordinates are used in translation devices for making the proper cut position adjustment.

In accordance with another aspect of the embodiment, a system is created including means for performing the method as outlined above.

In accordance with still a further aspect of the embodiment there is provided a system for cutting a cigar wrapper from a natural tobacco leaf having internal color variations and variations and defects detectable by light rays. The system comprises means defining a visible light conducting surface, means for supporting the leaf in a fixed, spread condition on the surface, means passing light rays through the surface and through the leaf whereby the rays pass through both the surface and leaf and have a light intensity at identifiable locations on the surface indicative of the visible light shielding efficiency of the leaf and surface at the various locations on the surface, and light intensity responsive means for scanning each of these locations in a preselected sequence. This scanning operation, by using a light passed through the leaf, can produce a profile or facsimile with accurate indications based upon light transmission of the actual leaf being processed. This system also includes first reading means controlled by the scanning means for creating a first signal when the light intensity of the passed light rays for one of the identifiable locations is above a light intensity and a second reading means controlled by the scanning means for creating a second signal when the light intensity of the passed light rays for the identifiable locations is below a second light intensity. Means are then provided for recording at least those identifiable locations wherein there is a transition to or from either the first or second signals. Means are then provided for selecting a wrapper cut position based upon the recorded locations and means for transferring the leaf on to a cutting platen at a selected location corresponding to the identifiable locations so that the selected cutting position can be identified and the leaf can be adjusted to the desired cutting position for cutting a wrapper based upon the previously selected wrapper cut position. In this manner, the profile or facsimile created by passing light through the leaf is used for selecting the cut position based upon recorded readings of the various light intensities across the surface of the leaf. Of course, this process can involve the selection of two or more cut positions for any leaf being processed.

In accordance with another aspect of the embodiment, there is provided a means for constructing a digital profile or facsimile representative of the energy intensity at the previously mentioned identifiable locations and means for selecting the cutting positions based upon this profile or facsimile for subsequent cutting at a different selected cut position which remains coordinated with respect to the natural leaf.

In accordance with still a further aspect of the embodiment there is provided an apparatus for detecting the general outline and abrupt surface variations

0004170

of a flaccid sheet-like workpiece formed from at least a portion of a natural leaf, this apparatus comprises a foraminate, visible light ray conducting workpiece receiving element having a first workpiece supporting surface with an area and shape capable of receiving the workpiece in a spread condition and a coterminous second surface, means for creating a positive pressure differential from the first surface to the second surface whereby the workpiece is held against the first surface, a light source means on one side of the element for passing light rays through the workpiece receiving element and through the workpiece, with the transmitted light intensity on the other side of the element being indicative of the outline and surface conditions of the workpiece and a light sensitive transducer means for receiving the light rays on the other side of the element. This transducer means includes a plurality of light responsive transducers juxtapositioned along a given path and means for directing the light rays from only a finite linear portion of the workpiece receiving element to the given path. In this manner, the transducer means receives the light intensity along a given line of the workpiece. There is provided means for incrementally changing the finite linear position sequentially from position-to-position along the workpiece and means for reading the conditions of the transducers at each of these positions. In this manner, the profile or facsimile of the workpiece, such as a tobacco leaf, can be recorded based upon light passed through the leaf itself. This concept provides information regarding surface defects and discoloration of both surfaces of the workpiece together with various defects common to both surfaces of the leaf.

Various other aspects of the embodiment will become apparent from the following description thereof and the appended claims hereof. By using these various aspects of the embodiment, one or more cigar wrappers can be cut automatically from a natural tobacco leaf without manual manipulation of the leaf itself, as heretofore required for the processing of such wrappers.

One object of the present invention is the provision of a method, system and apparatus for producing cut profiles of a preselected shape from a natural leaf, which method, system and apparatus automatically locates the cut positions and positions the leaf relative to a cutter having the shape of the cut to be made.

Another object of the present invention is the provision of a method, system and apparatus, as defined above, which system develops a facsimile duplicating a remotely positioned leaf and surface defects thereon for location of cut positions.

Still a further object of the present invention is the provision of a method, system and apparatus, as defined above, which method, system and apparatus develops a leaf facsimile as a digital pattern in a memory for subsequent processing to locate cut positions.

Another object of the present invention is the provision of a method, system and apparatus, as defined above, wherein a facsimile having the cut contour is compared with the created facsimile of the leaf and its defects to identify one or more cut positions on the leaf.

Another object of the present invention is the provision of a method, system and apparatus, as defined above, wherein a whole leaf can be processed without previous stemming.

Still a further object of the present invention is the provision of a method, system and apparatus, as defined above, wherein the facsimile is created by shining a light through the leaf so that surface variations within the leaf itself and on both surfaces of the leaf can be simultaneously obtained for use in the leaf facsimile.

Yet another object of the present invention is the provision

0004170

of a method, system and apparatus, as defined above, that allows scanning of the leaf in one position to obtain the desired facsimile and cutting sections from the leaf in another position. In this regard, one surface can be used for obtaining the profile or facsimile and a second surface can be oriented with respect to the first surface to receive a leaf in a known position.

Still a further object of the present invention is the provision of a method, system and apparatus, as defined above, which optimizes the number of cuts that can be made in a leaf taking into consideration the major defects and edge outline of the leaf.

Another object of the present invention is the provision of a method, system and apparatus that produces cigar wrappers from a natural tobacco leaf without manual manipulation of the leaf and without stemming of the leaf.

These and other objects and advantages will become apparent from the following portions of the specification.

BRIEF DESCRIPTION OF DRAWINGS

FIGURE 1 is a top plan view illustrating a natural tobacco leaf from which the wrappers are to be cut;

FIGURE 2 is a schematic view illustrating the shape of the cigar wrapper to be cut in the preferred embodiment together with the encompassing profile or facsimile shape shown in dashed lines to be used in locating the cut position on the leaf shown in FIGURE 1;

FIGURE 3 is a top plan view of the leaf shown in FIGURE 1 with the cutter profiles located in cut positions;

FIGURE 4 is a block diagram showing schematically certain general features of the preferred system;

FIGURE 5 is a block diagram outlining control concepts employed in the preferred system of the present invention;

0004170

FIGURE 6 is a flow chart block diagram illustrating interfacing concepts between the computer and the programmable controller as shown in FIGURE 5;

FIGURE 7 is a schematic, side elevational view showing the scanning mechanism and the transfer arrangement employed in the illustrated embodiment of the present invention;

FIGURE 7A is an enlarged cross-sectional view showing certain features of the structure illustrated in FIGURE 7;

FIGURE 7B is a schematic representation of the light intensity transducer units used in the illustrated embodiment of FIGURE 7;

FIGURE 8 is a logic diagram illustrating in block diagram and logic diagram form the control system for the scanning operation to be employed in the structure as schematically illustrated in FIGURES 7, 7A and 7B;

FIGURE 9 is a schematic differential circuit which would create light intensity outputs as graphically illustrated in FIGURE 8;

FIGURE 10 is a chart showing an operating characteristic of the light intensity scanning arrangement employed in an embodiment of the present invention without the preferred modification as contemplated by the system as shown in FIGURE 11;

FIGURE 11 is a block diagram of the circuit for modifying the video output of the scanning unit to produce an output modified from that of FIGURES 8 and 9;

FIGURE 12 is a graph illustrating the operating characteristics of the circuit shown in FIGURE 11;

FIGURE 13 is a block diagram of the memory loading concept which could be employed in the system when raw data of all locations is used for creating a leaf facsimile;

FIGURE 14 is a logic diagram illustrating a system for creating transitions in the scanning operation for use as intermediate data in the preferred embodiment of the present system;

FIGURE 14A is a simplified logic diagram illustrating a transition data combining digital circuit employed in conjunction with FIGURE 14 and used in the preferred system;

FIGURE 15 is a block diagram illustrating a data transfer system which could employ the data developed by the structure illustrated in FIGURE 14 and digital circuitry for processing and inputting such data;

FIGURES 16 and 17 are data transfer circuits similar to that shown in FIGURE 15 but employing the data developed by the schematic digital circuit of FIGURE 14A;

FIGURE 18 is a block diagram illustrating the direct access memory loading concept used in the preferred system wherein transition data is loaded into separate memory units or areas;

FIGURES 19 and 20 are tabulations of direct memory entered data employed in the preferred system of the present invention;

FIGURE 21 is a schematic view illustrating a natural tobacco leaf and certain scanning concepts employed in the preferred scanning concept of the system;

FIGURE 22 is a vectorized outline of the natural leaf shown in FIGURE 21 as employed in the preferred processing concept of the system;

FIGURES 23, 24 and 25 illustrate further concepts used in processing data employed in the preferred system;

FIGURE 26 is an outline of the computer program steps employed in the preferred system of the present invention;

FIGURE 27 is a top plan view of an embodiment of the present invention illustrating the various structural features of a system for locating the cuts on a natural tobacco leaf, transferring the tobacco leaf to a cutting platen, cutting the cuts from the natural tobacco leaf and conveying the cuts or wrappers to a successive position for processing;

FIGURE 28 is a schematic view taken generally along lines 28-28 of FIGURE 27;

FIGURE 29 is an enlarged cross-sectional view taken generally along line 29-29 of FIGURE 27;

FIGURE 30 is an enlarged plan view of the cutting head take generally along line 30-30 of FIGURE 29;

FIGURE 31 is a side cross-sectional view taken generally along line 31-31 of FIGURE 29;

FIGURE 32 is a side elevational view showing the cutting platen in the cutting position;

FIGURE 33 is an enlarged cross-sectional view taken generally along line 33-33 of FIGURE 27;

FIGURE 34 is a graphic view of the cutting table employed in the preferred mechanism of the present invention;

FIGURE 35 is a cross-sectional view of the cutting table as illustrated in FIGURE 34;

FIGURE 36 is a top plan view showing in more detail the cutting platen and cutting table employed in the preferred embodiment of the present invention;

FIGURES 36A, 36B are plan views illustrating the operating characteristics of the structure shown in FIGURE 36;

FIGURE 37 is a cross-sectional view of the digital motors in the structure illustrated in FIGURE 36;

FIGURE 38 is a cross-sectional view taken generally along line 38-38 of FIGURE 37;

FIGURE 39 is a schematic logic diagram illustrating operating characteristics of the structure shown in FIGURE 37;

FIGURE 40 is a schematic view of a modification of the illustrated preferred system showing certain features of the present invention;

FIGURE 40A is a schematic view showing a modified portion of the structure as illustrated in FIGURE 40;

FIGURE 41 is a further embodiment of the present invention wherein the scanning, cutting, loading and unloading of a natural leaf onto the cutting platen is done at angularly positioned locations and the cutting occurs on the same support structure as used during the scanning operation;

FIGURE 42 is a schematic view illustrating the grid coordination concept employed in the preferred embodiment of the present invention; and

FIGURE 43 is a simplified schematic view illustrating a modification of the invention as could be used in the schematic structure illustrated in FIGURE 41.

- 16 -

0004170

## DESCRIPTION OF INVENTIVE CONCEPTS, PREFERRED EMBODIMENTS AND MODIFICATIONS THEREOF

The present invention relates to a method, system and apparatus for cutting one or more profiles, such as cigar wrappers, from a natural leaf, such as a tobacco leaf. A natural leaf has veins, color variations, a stem in most instances, holes and various defects which are not to be included in the profile cut from the leaf. As a general description, the invention relates to the method, system or apparatus for accomplishing this primary objective by using a constructed profile or image of the leaf which depicts the defects, without using any marking arrangement. The system uses this constructe profile or image for selecting the cut position or positions in a manner generally optimizing the number of wrappers and the quality of wrapper taken from the available surface area of the leaf. The basic and total concept of this method, system and apparatus will be explained in detail. The appended claims set forth the inventive concepts relating to this total description which will be separated into areas for illustrating the various aspects of the method, system and apparatus contemplated.

## GENERAL CONCEPTS

### (FIGURES 1-6)

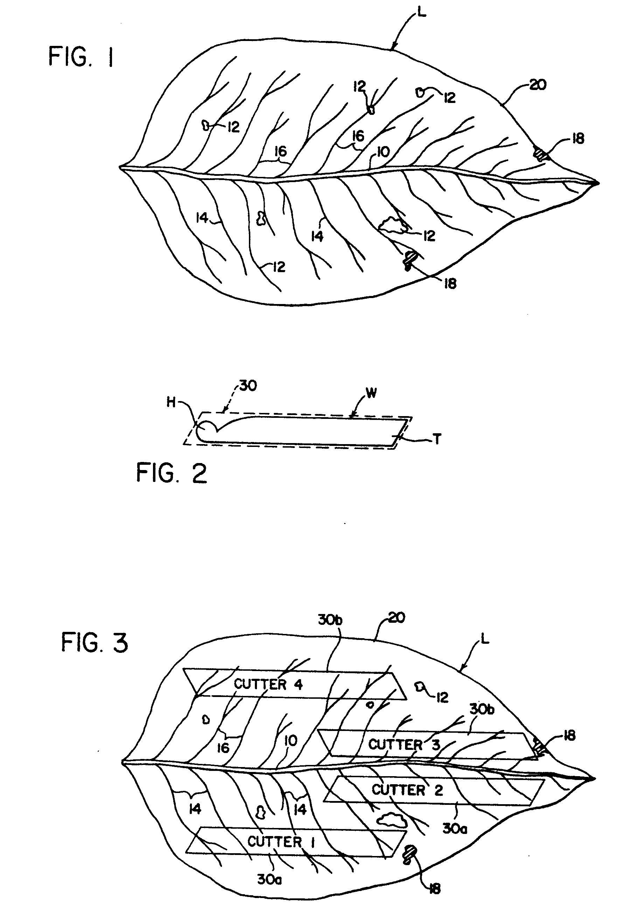

Referring now to FIGURES 1-3, a natural tobacco leaf L has known physical characteristics in that it is flaccid, pliable and has internal color variations, energy ray detectable variations and defects like holes, and dark or black areas. In addi the natural tobacco leaf has a top surface which is to be used as the exposed portion of the wrapper to be cut from the leaf. Natural leaf L is illustrated as including a center stem 10 whi extends in a somewhat undulating path longitudinally through
the leaf, a plurality of holes 12 of varying size, diagonally
extending right and left veins 14, 16, respectively, dark
areas 18 and an outline or edge 20. The dark areas 18 may be
on either surface of the leaf and are visible from the top
surface to a degree determined by their location. Indeed,
the dark areas may be internal of the leaf and present only a
slight color variation when viewed from the top surface of the
leaf. Even in that situation, the dark areas could be considered unacceptable for a wrapper of a cigar. Also, abrupt
surface color variations can occur as pronounced light areas
in the leaf which may be detectable from one or both surfaces
of the leaf and which may be undesirable for a cigar wrapper.
From the leaf L cigar wrappers W, as shown in FIGURE 2, are
to be cut by a cookie cutter type die or clicker die commonly
used for cutting of flat sheets in other industries. Wrapper
W has a precise shape which allows spiral winding of the wrapper around the binder and/or core of the cigar without producing wrinkles and surface defects. The wrapper generally
includes a head H and a portion T called a "tuck". Tuck T
is ultimately located in the end of the cigar to be lighted,
whereas head H is twisted and wrapped into the portion of the
cigar to be held in the smoker's mouth. These shapes are
critical and it should be free of dark areas, any holes and
heavy veins. The shape of wrapper W, which varies for
different length and shaped cigars, can be circumscribed by
a four-sided profile 30. The profile is different for left
and right hand wrappers and are configurations 30a, 30b,
as shown in FIGURE 3. The cutters are labeled CUTTERS 1-4.
In this illustrated embodiment the cutters outlined by the
shapes 30a, 30b represent cut positions of the same wrapper
shapes. In some instances, two or more wrapper shapes could be cut from a single leaf. For the purpose of describing the present system, the cutter to be used in the wrapper has the shape of wrapper W; however, the profile 30 or profiles 30a, 30b are used to locate the cut positions for the wrappers on the surface of leaf L in a manner to avoid any unwanted defects in the resulting wrapper within the confines of general profiles 30a, 30b. In producing wrappers W from leaf L the wrapper profile must be located on the leaf to avoid defects such as holes, the stem, dark areas, color variations of a given magnitude and other such contingencies to produce a wrapper having the quality set forth in the introductory portion of this application. In most arrangements for cutting the wrapper W from a natural tobacco leaf, the stem is removed and the leaf halves are booked or stacked according to the right hand vein arrangement or the left hand vein arrangement. The present system does not contemplate the stemming of the leaf; however, it could be stemmed and a half of a leaf could be processed in the present system. Throughout the rest of the discussion of the present system, wrapper profile 30 is used to locate the cut positions on leaf L of the cutter for a wrapper W since the placement of this profile at various non-conflicting locations will provide proper cut positions for the circumscribed actual wrapper configuration. In practice of the present system, the actual profile 30 or profiles 30a, 30b are slightly greater in size than the contour of the wrapper cutter so that the cut made by the cutter itself will not be at the edge of the leaf or closely adjacent a prohibitive defect to be excluded. In other words, the profile used in selecting the cut positions in accordance with the system herein described is slightly larger than the actual cutter profile used in making the wrapper cut in leaf L so that the cut is offset at least a small amount from the outline of profile 30.

In accordance with the system contemplated by the present invention, a profile or image of the leaf including the outline, stem, surface variations and defects is created and recorded and stored. Thereafter, the profile 30 is manipulated within stored profile or image to locate positions in which the profile 30 avoids unwanted defects and remains in the confines of the leaf. These selected cut positions are then used to control a cutting machine that positions the leaf and wrapper cutter in the desired relative position for a cutting operation to remove the wrapper from the area bound by profile 30 in its final selected position or positions.

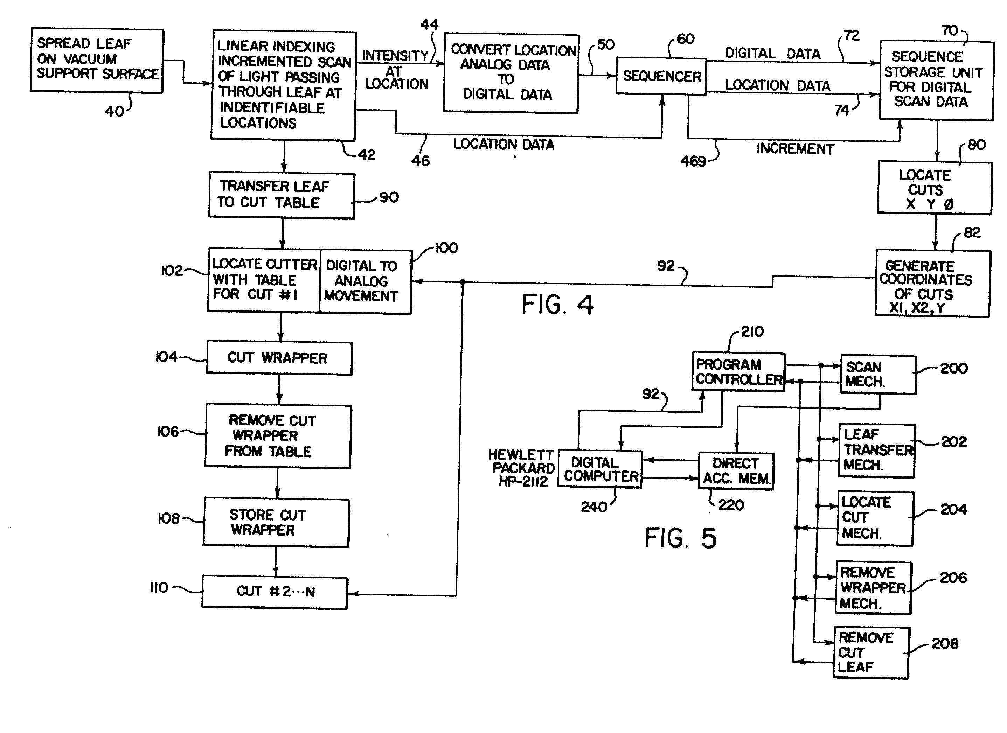

A general functional block diagram of the overall system is schematically illustrated in FIGURE 4. A description of this block diagram will provide general information regarding the basic concept of the system hereinafter described and can be used as an over view of the system of cutting wrappers W from desired positions on leaf L without requiring the previously used manual manipulation and without sacrificing quality of the resulting wrappers. In accordance with the functional block diagram, a natural leaf is spread onto a vacuum support surface which preferably is capable of transmitting light rays so that the rays pass simultaneously through the surface and through the leaf. These rays are read by a light intensity sensitive device which determines the light intensity at selected locations on the support surface. The total light intensity pattern provides a light intensity profile or image of the natural tobacco leaf supported in a spread condition on the surface. The concept of spreading the natural leaf onto a vacuum support surface for cutting is known and is represented by block 40. The concept of passing light through the leaf supported on a vacuum

0004170

surface is novel and is represented in block 42. As indicated by block 42, the leaf is indexed or moved incrementally in one direction (hereinafter called the Y direction) and the light intensities at selected areas of the leaf across another direction (hereinafter called the X direction) are recorded at identifiable locations identifiable by both X and Y positions on an imaginary grid on the vacuum surface. The intensity of the light passing through the leaf at these various identifiable locations which cover the total leaf as it has been moved are indicative of the contour of the leaf and color variation or defects including holes, dark areas, the stem, the veins and other surface color variations. By passing light through the leaf, variations on both surfaces are detected as are variations within the leaf which can cause discernible color variations that would be objectionable in a wrapper for a cigar. Block 42 is indicative of this scanning operation for obtaining light intensity at selected locations encompassing the total surface area of natural tobacco leaf L. The scanning operation creates a series of light intensity signals which are each analog voltages. One voltage signal corresponds to each scanned location and appears in line 44. Digital data indicative of the location of the scanned locations simultaneously appears in line 46. The analog light intensity signal in line 44 is converted to digital data indicating whether or not the scanned location has a light intensity value indicative of white, black or an intermediate color, termed "gray". Thus, each of the scanned locations on the leaf, which may be as small as desired and is known as a Pixel, will produce a light intensity, digital signal in line 50. The Pixel data will be white, black or gray. If desired, variations of gray could be obtained; however, such resolution is not required in the present system. The address data in line 46 identifies the location of the Pixel being
detected and transmitted in line 50. As can be seen, if
the leaf is segregated into a large number of identifiable
locations, or Pixels, which have known addresses (in the
X and Y directions) and known light intensity conditions,
such as white, black or gray, a profile of the leaf itself
can be constructed remote from the leaf. For illustrative
purposes, a sequencer 60 is indicated as transmitting the
digital information regarding the color of the Pixel and the
location of the Pixel to any type of storage unit 70 by lines
72, 74. The particular storage arrangement will then contain
a matrix indicative of the profile of the leaf including an
outline and defects. This data matrix storage unit 70 is
indicated to be a sequenced unit incremented by line 469 for
each Pixel in the X direction. In practice, storage unit 70
is a direct accessed memory usable by a digital computer in
accordance with known computer practice. The indexing by
each X Pixel will cover all Y indexes in sequence. Proceeding further into the schematic representation of the general
system as shown in FIGURE 4, cut locations or positions are
determined as represented by block 80 using the stored digital
data in storage unit 70. This can be done in a variety of
computer arrangements which will be described in more detail
later. Basically, a digital representation of the cutter
profile 30 is compared to the stored digital profile of leaf
L in storage unit 70 and these two profiles are shifted
until the digitized profile 30 does not include a defect, such
as a hole, edge of the leaf, or undue color variations. After
one cut position is located, a next profile 30 is also shifted
with respect to the digitized leaf profile or image in storage
unit 70 to locate still a further cut position which avoids
the defects set forth above. This process is continued until
the digitized representation of profile 30 is set to the extent possible to obtain the desired number of cuts in leaf L. The various cut locations so located by shifting the digitized profiles 30 within the digitized leaf profile of storage unit 70 are in the form of X, Y and $\emptyset$ corresponding to the X, Y location of a selected point on a line of profile 30 and the angle this line makes with the scanned Y direction.. The X, Y and $\emptyset$ coordinates are then converted to usable cut coordinates represented as X1, X2 and Y by a coordinate arithmetic conversion. This conversion is determined by the usable data necessary to obtain the same X, Y and $\emptyset$ location on the cutting machine when a linear converting mechanism is used for purposes to be explained later. Arithmetic conversion to cut coordinates usable in the specific machinery contemplated for actually making the cut in the leaf is represented by function block 82. After the scanning operation schematically illustrated as block 42 has been completed, leaf L is transferred to a cutting platen or table. This process is represented by functional block 90. After the leaf has been transferred to the cut platen, the arithmetically generated coordinates from generator 82 are directed through an interface indicated generally as line 92 to a digital-to-analog movement mechanism 100. This mechanism shifts the cut table carrying the leaf to a known cut position determined by coordinates from interface 92. As will be explained later, the movement by mechanism 100 is translated along three axes corresponding to X1, X2 and Y coordinates. The moving to a cut position is represented by block 102. The transfer of leaf L to the cut table is in an oriented position and the cut table is a vacuum surface which holds leaf L in this oriented position. The coordinates from generator 82 operate on the table as they would on the scanning surface. After the leaf and table have been shifted to the first cut position, the wrapper cutter is shifted downwardly to cut a cigar wrapper from the leaf in this first cut position. This action is represented by functional block 104. The cutter is then raised to remove the wrapper from the leaf, which leaf is still held on the table by vacuum. The wrapper is held by a vacuum means as the cutter moves upwardly. This removes the wrapper from the cutting table as represented by functional block 106. Thereafter, the wrapper is removed from the cutter by a vacuum transfer arrangement in accordance with known wrapper handling procedures. This functional step is illustrated as block 108. Interface 92 then causes a recycling of the cutter table to the second cut position and a second wrapper is cut, removed and stored for subsequent use. This procedure is continued until all the cuts located within leaf L have been made. Thereafter, the next leaf is processed.

In accordance with the preferred embodiment, while the cutting operation is taking place on one leaf, a second leaf is being spread on the vacuum support surface and scanned and the cut positions are being located and the coordinates X1, X2 and Y are being created. Thus, a tandem arrangement is contemplated wherein the scanning is taking place on one leaf while the cutting operation is taking place on a previous leaf. To facilitate this dual operation, a general control system, as shown schematically in FIGURE 5, is employed in the preferred embodiment. The system includes, as basic machine elements, a scanning mechanism 200, a leaf transfer mechanism 202, a locate and cut mechanism 204, a wrapper removing mechanism 206 and a mechanism 208 for removing the spent leaf after all the cuts have been made from the leaf. All of these mechanisms, which will be explained in more detail later, are controlled in accordance with a standard practice by a commercially available programmable controller 210, such as one using an
Intel 8080 microprocessor. The sequence of mechanical operations are conducted in accordance with the program of the
programmable controller in accordance with standard practice
in machine control operation. The scanning mechanism 200
provides information to a direct access memory 220 which has
a two-way communication with a standard digital computer 240
that locates the cut positions, converts them to X1, X2 and Y
coordinates and provides the coordinates to the controller
210. A two-way communication with the programmable controller
is provided to load the cut positions into the programmable
controller when the programmable controller is ready to accept
the cut coordinates for processing a scanned leaf L. The
control and function of information provided in FIGURES 4 and
5 is illustrative in nature to show the general process being
performed in accordance with the novel system and a general
control arrangement which allows the various mechanisms to be
operated to cut wrappers from leaf L. This use of a controller
210 reduces computer time and software and provides a standard
type of machine control for the various mechanisms used to
perform the sequence of events set forth generally in FIGURE
4.

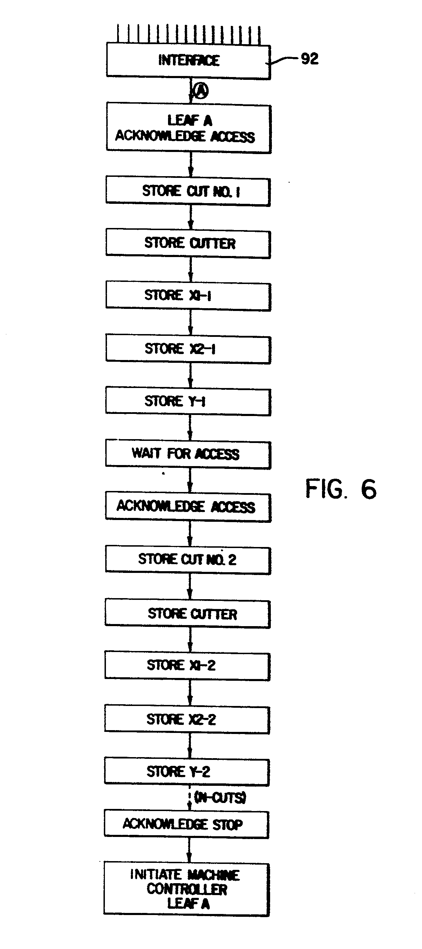

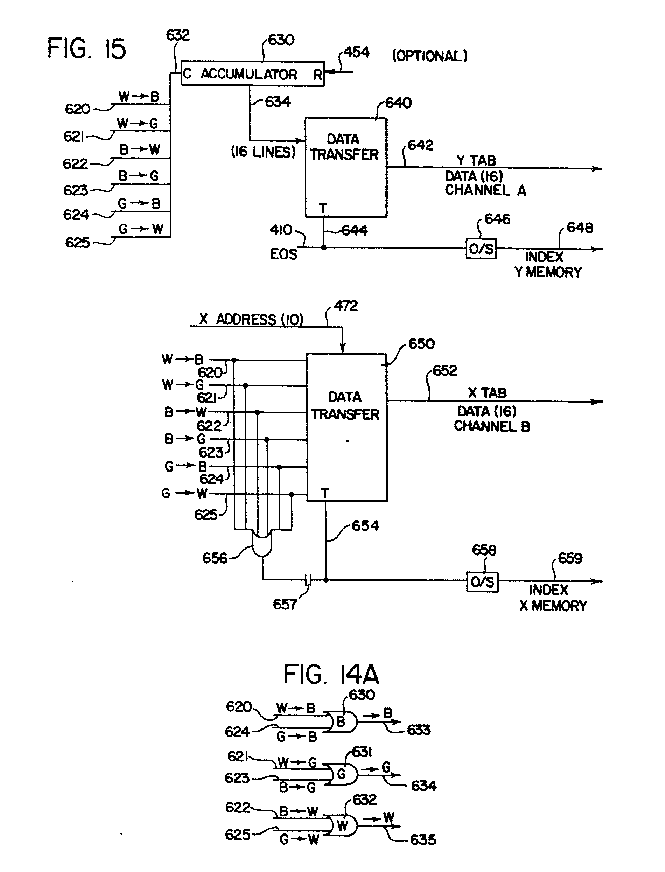

Referring now to FIGURE 6, this figure illustrates, somewhat generally, the interface 92 between the programmable
controller 210 and the digital computer 240 as represented in
FIGURE 5. Also, the information from computer 240 is illustrated in FIGURE 13. As a first step, the programmable
controller 210 acknowledges that it desires access to the
digital computer 240 for data regarding the cut coordinates
for leaf designated as "leaf A". Thereafter, the programmable
controller obtains and stores the number of the cut (1, 2 ...
n), the particular cutter (cutter 1, 2, 3, etc), the coordinates X1, X2 and Y for the first cut. It is possible to use
different cutters to obtain different wrapper sizes. For that reason, the particular cutter to be used is inputted to the programmable controller. In addition, as noted in FIGURE 3, there are left and right hand wrapper cutters to accommodate the different vein configurations. Thus, if a cut is to be made from one side of the leaf, one cutter is selected, whereas another cutter is selected for a wrapper from the other side of the leaf. Because of this, the programmable controller receives information regarding which cutter is to be used, which cuts are to be made and where the cut is to be made on the leaf. Thereafter, the programmable controller waits for access again from the computer, acknowledges access and receives the information regarding the second cut. This continues until the programmable controller receives information regarding all n cuts to be made in leaf A. At that time, the programmable controller acknowledges that all information has been received with respect to leaf A and proceeds to initiate the controller for processing leaf A. After leaf A has been processed, the programmable controller then receives information regarding the cut positions and cutters from the digital computer for making the cuts in the next leaf. The cutting operation is proceeding as the scanning operation for the next leaf is proceeding. FIGURES 4, 5 and 6 are to be taken together to illustrate one arrangement for accomplishing the system contemplated by the present invention. The details of the system will be hereinafter set forth. However, it should be appreciated that there are several machine control arrangements and computer arrangements which can be employed in practicing the method and system contemplated by the description herein.

<div align="center">

SCANNING AND CREATING DIGITAL FACSIMILE DATA

(FIGURES 7, 7A, 7B and 8-12)

</div>

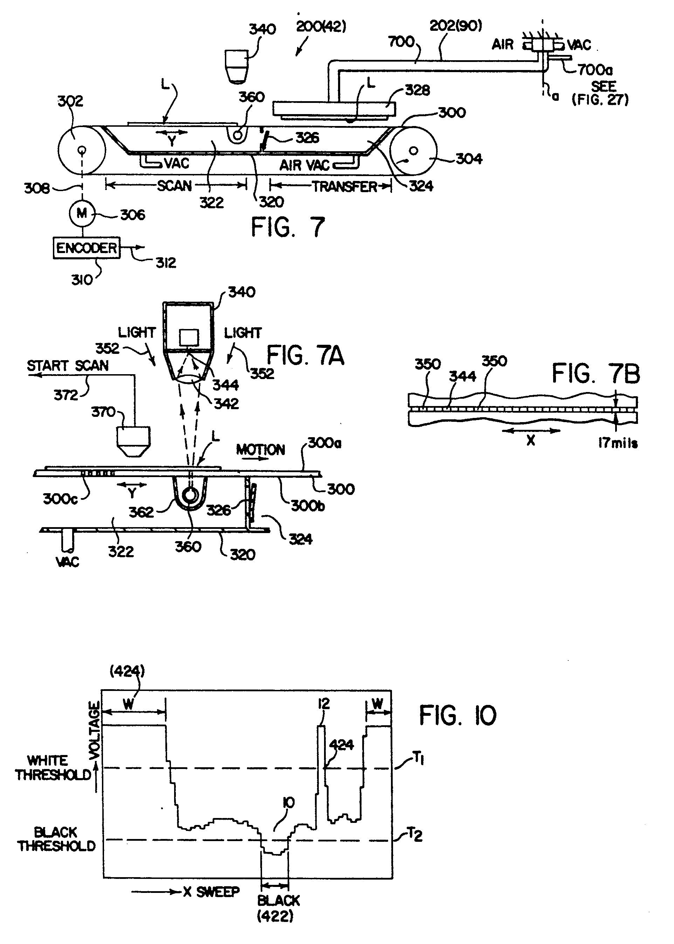

As explained with respect to the general description above, a system in accordance with the present invention involves an arrangement for obtaining digital information regarding the light transmitted characteristics at various identifiable locations or Pixels (X, Y locations) on a natural tobacco leaf L for processing of this information to select available cut positions for one or more cuts to be made in the tobacco leaf in the process of forming wrappers for cigars. As indicated in the block diagram of FIGURE 4, used for illustrating certain basic concepts of the system under consideration, block 42 contemplates a scanning mechanism schematically represented as mechanism 200 in the control concept block diagram shown in FIGURE 5. A variety of mechanisms 200 could be employed for the scanning operation to produce the desired digital information. One of the basic concepts of the present system is the generation of a signal, preferably digital, indicative of the surface or color condition of selected, known locations or Pixels on the tobacco leaf, which Pixels can be combined and processed in a manner generally described above to give a representative facsimile, image or profile of the leaf including both color variation and defects such as holes and dark areas and stems. This facsimile, profile or image is then used to graphically or arithmetically locate the particular cut positions for subsequent cutting of wrappers from the natural leaf. To produce digital information or data regarding the surface condition or color variation at locations on leaf L, mechanism 200 is used. This mechanism is schematically illustrated in FIGURES 7, 7A, 7B, 8 and 9 which show the preferred scanning operation. In accordance with this illustrated embodiment, there is provided a continuously driven belt 300 movable around spaced support rolls 302, 304 and having an upper surface 300a and a lower or inner surface 300b. For the scanning operation, the belt is transparent or translucent so that it can transmit light rays through the belt for a purpose to be described later. In practice, this belt is translucent and formed from a material known as "Revo" which is a
clear nylon belting supplied by L. H. Shingle Company and has a
general thickness of 0.04 inches. This standard nylon belting
is provided with small perforations 300c extending through the
belting to develop a positive pressure differential adjacent
upper surface 300a when a vacuum is created adjacent lower surface 300b. The use of the vacuum at surface 300a holds leaf L
in a manually or machine spread condition on leaf receiving surface or support surface 300a in the "scan" position shown in
FIGURE 7. To drive the belt in a clockwise direction, as shown
in FIGURE 7, there is provided an appropriate electric motor 306
connected to roll 302 by a drive mechanism schematically represented as dashed line 308. An encoder 310 provides pulses in
output line 312 when motor 306 has driven belt 300 axially a
preselected distance. In practice, this preselected distance
is 0.05 inches. Consequently, an encoder pulse is provided after
each movement of 0.05 inches of belt 300 in the Y direction indicated in FIGURES 7 and 7A. Below the belt in both the "scan" and
"transfer" positions there is provided a vacuum box 320 of somewhat standard design in the cigar making industry. This vacuum
box substantially traverses the "scan" and "transfer" positions
of the upper run of belt 300 between rolls 302 and 304. If the
leaf is to be placed on belt 300 in the lower run, a vacuum box
could be provided along this run and roll 302 could be a standard
vacuum roll. Adjacent the scan position there is provided a
vacuum chamber 322 which is maintained at a vacuum by an appropriate
external vacuum source. In the transfer position, there is provided
a chamber 324 which combines with chamber 322 to form the total

box 320. A pressure sensitive flapper valve 326 separates chambers 322, 324 to allow selective transfer of the leaf by a mechanism 202, which will be explained later. The leaf is to be removed from upper surface 300a of belt 300 in the transfer area after it has been scanned. Transfer chamber 324 can be provided with a vacuum or with pressurized air. After a leaf has been scanned, it is moved to the transfer position. At that position chamber 324 is pressurized. This allows the scanned leaf to be grasped by a vacuumized transfer head 328. This head has a lower vacuumized leaf receiving surface that holds the leaf L in an oriented position and transfers it to the cutting table in a manner to be described later. Thus, to transfer the leaf from belt 300 to transfer head 328 pressure is applied to chamber 324 and vacuum is created adjacent the lower surface of transfer head 328. This transfer head and procedure is used often in the tobacco industry to transfer tobacco products from a belt to another structure. When pressure is applied to chamber 324, valve 326 closes to prevent loss of vacuum in scanning chamber 322. Of course, other arrangements could be used for holding leaf L by vacuum onto a scanning surface while the surface is being moved and then for removing the leaf from the scanning surface; however, the arrangement illustrated in FIGURE 7 is now contemplated for use in the present system. Belt 300 is stopped momentarily for leaf transfer.

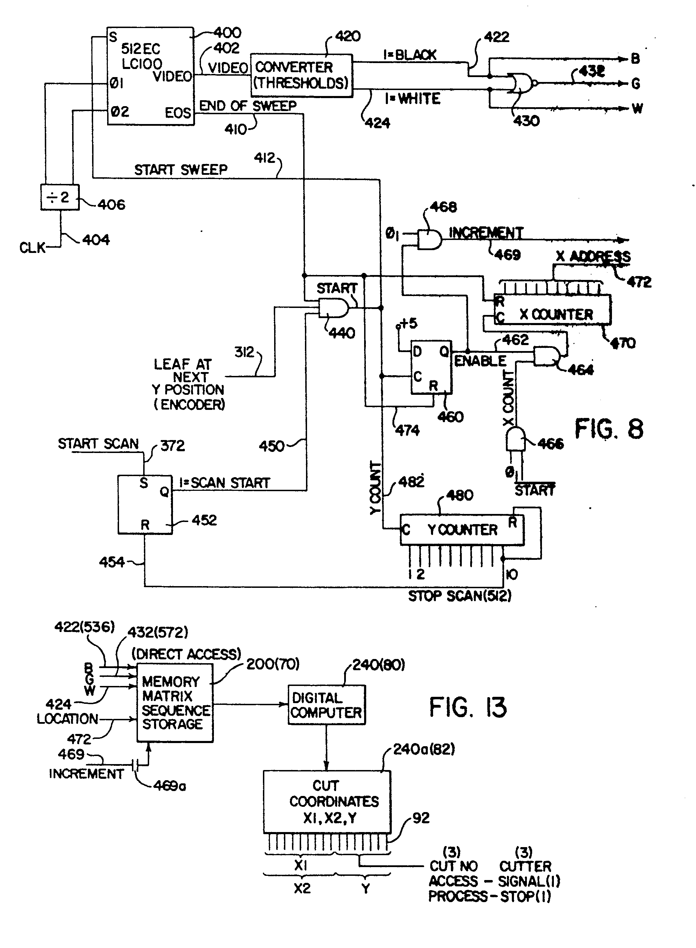

In accordance with the preferred embodiment of the present invention, scanning device 200 includes any appropriate mechanism for identifying the color content or the light intensity characteristics at various preselected locations or Pixels across the face of tobacco leaf L at X and Y positions. In practice, the data creating mechanism is a scanning head 340 which simultaneously records light intensity at spaced locations across belt 300 in a direction, identified as the X direction in FIGURE 7B. The X direction is generally orthogonal to the longitudinal or Y direction shown in FIGURES 7 and 7A. In essence, the scanning head 340 receives light rays along a selected Y position which is indicative of the light intensity veiwed at given spaced positions across the leaf. The term "scanning" as used in conjunction with mechanism 200 and head 340 means that these positions are read in series although they may be simultaneously detected. The scanning feature is provided by the circuit shown in FIGURE 8. The light intensity at most locations in both the X and Y directions of leaf L is measured and an output is created representative of this light intensity. Of course, certain approximations are acceptable. Locations in the X direction are viewed by head 340 at spaced lines or positions in the Y direction. This can create certain small bands of undetected areas between adjacent Y positions. This feature is minimized by shifting or moving the scanned position only a small distance in the Y direction. This will provide sufficient digital information or data for identifiable locations on the surface of leaf L to create leaf profile for location of the profiles 30. In practice, a LC 100 512 EC Reticon detecting or scanning head 340 is employed. This type of unit is commercially available from Reticon Corporation of Sunnyvale, California. In this type of mechanism, a lens 342 directs light rays from the surface of leaf L into an elongated aperture 344. By using the lens, the view distance in the X direction at the leaf area is focused onto 512 transversely spaced light intensity sensing units 350 each having a width approximately 2 mils. Consequently, the light profile in the X direction at a given Y position is focused on and changes the charged characteristics of the light intensity sensitive units 350 schematically illus-
trated in FIGURE 7B.   In this manner, light intensity at 512
different indexed or shifted positions in the X direction of
belt 300 are read simultaneously by the separate units 350.   This
covers the total leaf width and much of surface 300a.   The Reticon
unit uses a clocking pulse to step a shift register which allows
serial reading of the light intensity data of each 512 units
350.   This serial output is an analog video output as described
in FIGURE 8.   The voltage of this video output is an analog representative of the light intensity exposed to each of the units 350
spaced in the X direction at a given Y position.   The setting of
units 350 in a given Y position is not scanning; however, the output from the Reticon unit is a scanning function since it reads
successive cells or units 350 and outputs them in series as representative of the light intensity condition viewed by the units
across a given Y position.   After all units 350 have been read,
scanning unit 350 is ready to receive the light intensity at selected positions across the next adjacent Y position of leaf L.
To do this, belt 300 moves leaf L by motor 306.   As will be explained later, encoder 310 produces a signal in line 312 which
indicates that the units or cells 350 are now ready to read the
light pattern across the next successive longitudinally spaced
position on surface 300a which carries and supports leaf L.   By
progressing the belt 300 in the Y direction, the output of the
Reticon sensing head 350 reads the identifiable transverse locations on surface 300a at the various orthogonally spaced positions
in the Y direction.   The spacing between the units 350 and leaf L
is such that a distance of approximately 12.5 inches is focused
by lens 342 onto the 512 light sensitive units.   This provides a
spacing in the X direction of approximately 0.025 inches as compare to a spacing of approximately 0.05 inches in the Y direction
as determined by the adjustable setting of encoder 312. As
is known, the 512 units or cells 350 are approximately 2 mils
in width; therefore, the spacing of the units from the leaf
and the lens produces a scan or viewing field in the X
direction of slightly over 1:10. Other arrangements could be
used to change the viewed field across leaf L. The setting
of unit 340 to provide a unit or cell 350 for each approximately 0.025 inches has proven satisfactory and provides an
identifiable location, or a Pixel, which is satisfactory in
size. The Pixel has Y direction dimensions of approximately
0.50 inches to give good resolution for the ultimately constructed image or profile of the leaf L. The output of the
Reticon unit for each of the Pixels in the X direction is a
video signal having a voltage level indicative of the sensed
light intensity at the individual cells 350 being read by
the Reticon unit in accordance with standard practice for
this type of unit. As so far described, scanning head 340
has produced an analog signal for each of 512 positions
across the surface 300a of belt 300 for a given location of
the belt in a Y direction. As the belt is moved by motor 306
to a next position creating an encoder output in line 312
this process of providing output analog signals for 512 positions in the X direction is repeated. This action is continued until the total leaf has been processed. In practice,
there are 512 Y direction positions to combine with the 512 X
direction positions. Since the Y direction positions are
substantially twice as great as the X direction positions, the
identifiable locations are more highly resolved in the X direction. The surface area on belt 300 being viewed by scanning
head 340 measures approximately 12 inches across and about 25
inches in length. This is sufficient to encompass natural
tobacco leaves of the type used in producing wrappers for cigars. This monitored field of view of course can be changed by the optics of the scanning operation and by changing the number of Y direction positions used to complete a total scan of the surface 3(0a carrying a leaf L. The belt can move continuously at a rate allowing Pixel reading.

To provide discernible light intensity for viewing by head 340, it is possible to light the leaf on belt 300 by appropriately spaced lights 352 schematically illustrated as arrows in FIGURE 7A. These lights can be at angles to accentuate such surface conditions as veins or can be perpendicularly directed toward the leaf surface. In any instance, this front lighting arrangement does not allow head 340 to detect certain surface defects, such as discoloration and defects within the leaf itself and certain color variation on leaf surface. To provide a more accurate light intensity profile of the total leaf, the preferred embodiment of the system uses a back lighting arrangement wherein light rays are passed simultaneously through belt 300 and leaf L. This procedure produces high resolution light intensity profile at the detecting cells 350. This back lighted profile is more nearly indicative of the actual condition of the surface of the leaf, together with certain conditions which may be within the leaf or on the opposite surface of the leaf. Thus, one aspect of the system, as contemplated for use in practice, is back lighting of the leaf to pass visible light rays or other detectable energy rays through both the belt 300 and leaf L. In accordance with this aspect of the novel system, there is provided a transversely extending apertured fluorescent light 360, with the elongated apertured window of the light extending generally parallel to the aperture 344 controlling light flow to the units or cells 350. This aperture in unit 340 is approximately 17 mils in width. The light rays from the apertured fluorescent light 360 are directed through belt 300 and leaf L, if the leaf is over the light, and into aperture 344. An apertured fluorescent light for use in the system is a stock item available from various sources and has an opening of approximately 30° and a reflective surface around the remainder of the interior surface of the light. This gives a directed light source aligned with the aperture 344. Light 360 is fixed with respect to scanning head 340 by an appropriate mounting arrangement including a shield 362. This shield further restricts the deflection of light from the area of the belt to be illuminated. In FIGURE 7A, an appropriate device, such as a photocell 370, is used to detect the leading edge of leaf L as it is conveyed by belt 300. This photocell creates a "start scan" signal in line 372 which will be used in the circuit illustrated in FIGURE 8 for the purpose of starting the scanning operation.

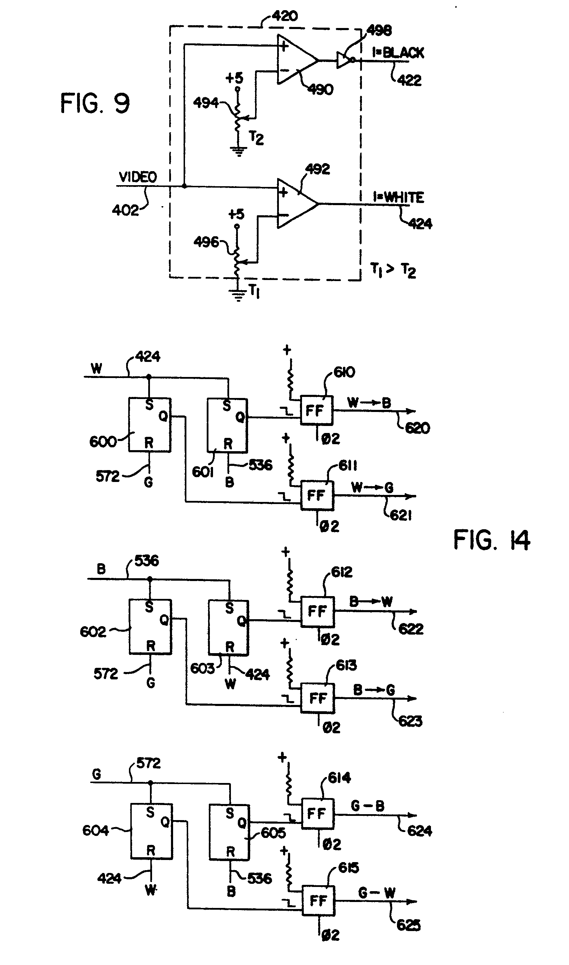

Referring now to FIGURE 8, a general scanning and output logic diagram for obtaining digital output facsimile data from the operation of scanner head 340 is illustrated. In this illustrated logic circuit, the standard Reticon reading device 400 is used to provide a series of analog data bits on line 402 which correspond to the X Pixels which are outputted in series by an external clocking pulse on line 404. These clocking pulses are divided by an appropriate divider 406 to produce two input clocks $\emptyset1$, $\emptyset2$ for controlling the scanning operation of the sequential reading device 400. This sequencing device employs a shift register to read each of the different units or cells 350 and to output an analog voltage on line 402 for each cell. At the end of a sweep across the cells, there is logic 1 produced in the end of sweep (EOS) line 410. The clock at line 404 may have a variety of frequencies; however, in practice it is in the range of 100 Kilohertz to 1 Megahertz according to the speed of the output signal required. Since a subsequent sweep is controlled by movement of the belt 300, as shown in FIGURE 7, a rapid sweep through the 512 Pixels is employed. A conversion circuit 420, best shown in FIGURE 9, produces a digital logic 1 in line 422 when the light intensity of a Pixel being read produces an analog voltage in line 402 less than a preselected voltage level. This logic 1 is indicative of a "black" condition for a Pixel. In a like manner, if the analog voltage of a Pixel is above a certain voltage level, which is indicative of a white condition, such as no leaf or holes in the leaf, a logic 1 appears in line 424. This is termed a "white" Pixel condition. A white or black condition indicated by the logic in lines 422, 424 control NOR gate 430 which produces a "gray" digital signal in line 432 when there is neither a black or white signal. Thus, a Pixel being read produces either a black, white, or gray digital output signal indicative of the light intensity at the Pixel. The gray signal is generally indicative of the natural color of the leaf being exposed to a cell 350. At the end of each sweep, a logic 1 in line 410 stops the video output at line 402. The sequencer 400 is initiated for a subsequent sweep by a logic 1 at the start terminal S. To create a logic 1 in the start line 412, there is provided an AND gate 440 having three inputs. The first input 312 is the output of encoder 310 shown in FIGURE 7. A signal appears in line 312 when the leaf is in the next Y position for a subsequent scan in the X direction. The second input to gate 440 is line 410 which is enabled when a prior sweep has been completed. The third input is line 450 which is a logic 1 during the scanning operation. A logic 0 in line 450 prevents scanning. A variety of circuits could be used for controlling the logic of line 450; however, in the illustrated embodiment, a flip-flop 452 is set by a logic 1 in line 372 from the photocell 370 shown in FIGURE 7A. Thus, when a leaf is moved by belt 300 into the scanning position, a logic 1 appears in line 372. This sets flip-flop 452 to enable gate 440. After the desired number of positions in the Y direction have been processed, in practice 512, a logic 1 appears in line 454. This resets flip-flop 452 and shifts a logic 0 into the line 450. This disables gate 440. Sweep start signals in line 412 can not be created until the next leaf sets flip-flop 452. Consequently, at each Y position a logic 1 is created at the output of gate 440 by line 312, if a leaf is being scanned. This starts the next X sweep. A logic 1 in line 412 not only starts a sweep, but also clocks flip-flop 460 to produce an enable signal in line 462. This enables X counting gate 464, controlled by gate 466, and enables increment gate 468. This latter gate is optional for a purpose to be described later. A logic 1 in line 412 up count Y counter 480 by pulsing Y count line 482. All of these functions are caused by a signal in line 312 as long as gate 440 is enabled by lines 410 and 450. As soon as a sweep has started, a logic 0 appears in the line 410 which inhibits gate 440 until the X sweep has been processed.

During an X sweep, output pulses are created in lines 422, 424 or 432. With each pulse, gate 466 clocks gate 464 to up count X counter 470. This produces the X address of the Pixel being read at the output of X counter 470. At the end of the X sweep, not only is gate 440 enabled, but X counter 470 is reset for the next sweep and flip-flop 460 is reset by line 474. This reset produces a logic 0 in the enable line 462 to disable counting gate 464 and incrementing gate 468. When Y counter 480 reaches a count of 512 the Y counter is reset. Line 454 resets flip-flop 452 to indicate that the scan is completed. Flip-flop 452 then prevents further scanning until the next leaf is available for processing.

In operation, for each sweep, the light intensity value of the Pixel and the X address of the Pixel is available at the output of the circuit shown in FIGURE 8. This information is available for each sweep until the Y counter has indicated that the desired number of X sweeps has been completed. At that time, the sweep circuit is deactivated until the next leaf is available for processing. The Y counter could be used to input the Y addresse for any given Pixel. In other words, the X address in lines 472 from X counter 470 could be combined with the output of Y counter 480 to give not only the X address for the Pixel, but also the Y address. The Y address is not generally required in the illustrated embodiment of the invention because it is sequenced in series.

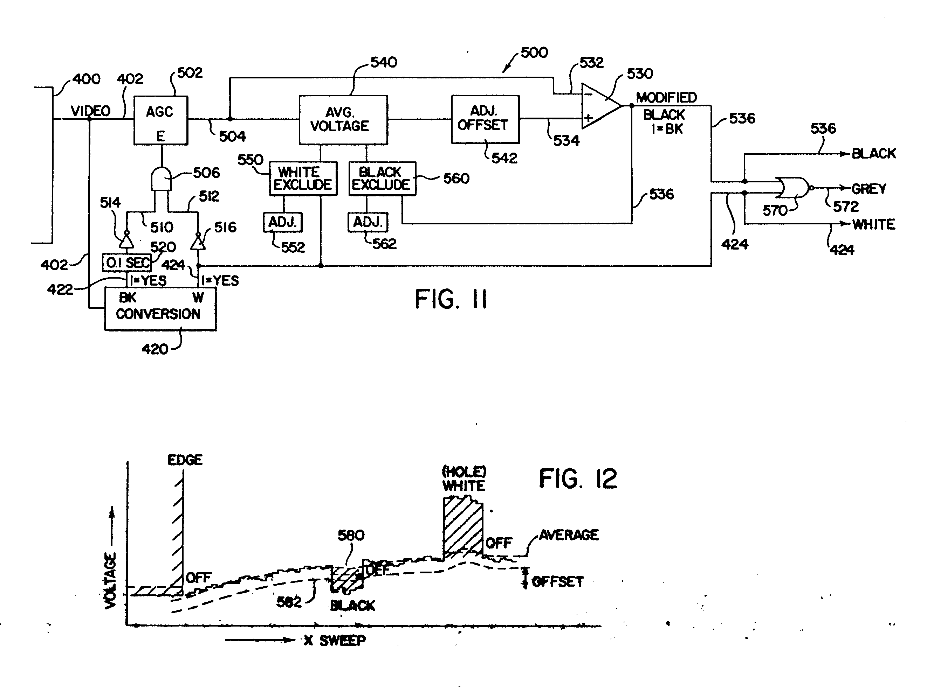

Referring now to FIGURE 9, the converter 420 is schematically illustrated as including two differential amplifiers 490, 492 poled as shown and having voltage adjustable rheostats 494, 496 to control the respective negative terminals. An inverter 498 inverts the output of amplifier 490 to produce a logic 1 in line 422 when a condition designated as "black" by rheostat 494 has been reached. Other arrangements could be used to convert the analog video signal in line 402 to a digital logic indicative of the black or white conditions. Referring now to FIGURE 10, this is a graph of a single X sweep across the leaf at a selected Y position. For illustrative purposes, the leaf has a center stem 10 and a hole 12. It is noted that opposite edges of the leaf are indicated by a white signal in line 424. The black stem is indicated by black signal in line 422. The hole 12 is represented by a white signal in line 424. Inbetween these extreme positions, logic 1 appears in line 432

This provides the general profile for the remainder of the leaf which does not exhibit either a drastically white or a drastically black condition. A sweep as illustrated in FIGURE 10 will be repeated 512 times for each leaf L and the information provided during each sweep can be used to create a profile of the leaf indicating the defect areas which should be avoided when locating a wrapper cut position on the leaf.

In practice, it was found that the procedure for obtaining black and gray Pixel information by comparing the light intensity with a fixed value for determining black presented some difficulties, especially when the color of the leaf changed or the conditions of the light or cells 350 varied. Consequently, in accordance with another aspect of this system a modified black signal is obtained by comparing the Pixel light intensity with a controlled average voltage. This average voltage uses the analog signals at line 402 for a majority of the Pixels. By comparing an analog Pixel voltage with an average voltage for prior Pixels, a black signal is recorded when there is in fact a substantial black condition compared with the operating conditions of the scanner and the color of the leaf. A circuit developed for obtaining this modified black signal is schematically illustrated in FIGURE 11. In this figure, the black modification conversion circuit 500 includes an automatic gain control, or amplifier, 502 which amplifies the analog Pixel voltage from line 402 and directs it to line 504. The automatic gain control or amplifier 502 is not needed in certain instances; therefore, it is enabled only when required by a gate 506. This gate has inputs 510, 512 connected by inverters 514, 516 with the output lines 422, 424, respectively, of conversion circuit 420 previously described. If the previously described conversion circuit produces a white signal, a logic 0 is directed to gate 506 and amplifier 502 is not activated. If a black signal is created in line 422 the automatic gain control or amplifier 502 is deactivated and held deactivated for a time delay indicated by the block 520. This time delay retains the black signal for a set time, which is 0.1 seconds in practice. In other words, gate 506 is deactivated whenever a white signal is created by circuit 420 and is held deactivated whenever a black signal is received. This allows the automatic gain control to be inactive when the sweep is mostly white, such as when there is no leaf or only a minor portion of the leaf is being detected by the sweep in the X direction. In this manner, the averaging concept does not become over weighted in a white voltage direction.

A differential amplifier 530 has inputs 532, 534 and an output 536 which receives the modified black signal. The voltage at input 534 is determined by the accumulated average of the Pixel voltage in line 504 as averaged by circuit 540. This is a capacitor averaging circuit in practice. The voltage of the capacitor averaging circuit is offset downwardly by an appropriate offset circuit 542. Consequently, the voltage at the input 534 is the average of the Pixel voltages, but offset downwardly for a reason to be explained later. To prevent the Pixel average voltage captured in circuit 540 from being distorted by white signals and black signals, there is provided a white exclusion circuit 550 which is adjustable as indicated by circuit 552. In a like manner, a black exclusion circuit 560 is provided with an adjustment circuit 562. Circuit 540 receives no signals when gate 506 is disabled because of mostly white being recorded. The circuit 550 removes those white areas which are not too large, such as a small hole.

The modified black output line 536 and line 424 are directed to NOR gate 570 to produce a gray Pixel data in line 572
when there is no white or modified black signal. During normal
X sweeps across the leaf, the amplifier 502 is operative. It
is only inoperative when there is a prolonged period of white.
This removes from the averaging function the predominantly
white area of surface 300a surrounding the leaf being scanned
or the white area of a large hole. As shown in FIGURE 12,
averaging circuit 540 captures an average voltage indicated
basically by the line 580. This average voltage line excludes
the black portions and white portions indicated by the legend
OFF either by disabling amplifier 502 or by exclusion circuits
550, 560. Offset circuit 542 offsets the average voltage
downwardly to a level shown as line 582 which is the voltage
used as a comparison voltage for the Pixel voltage in line
532 to determine a modified black output. By offsetting the
average voltage downwardly, only light intensities which are
lower than the offset average reference voltage in line 534
(line 582 of FIGURE 12) will produce a black signal. A white
signal is produced in accordance with the conversion circuit
420 as previously described. By using the circuit as shown
in FIGURE 11 and graphically depicted in FIGURE 12, a more
accurate color profile in the X direction is obtained. As
can be seen, the average voltage from a prior X sweep remains
in the circuit 540 for the starting point of the next sweep.
In this manner, if a group of dark leaves are being scanned,
black defects can be detected. In such an instance, a fixed
data voltage for detecting the black areas could indicate
the total leaf is black. Of course, it would be possible to
adjust the fixed black data for darker leaves or changes in
the response of scanner 340 to overcome some of this difficulty; however, the circuit 500 of FIGURE 11 automatically
makes appropriate compensations. FIGURES 9 and 11 could be
modified to give more than one shade of gray if needed.

As so far explained, the scanning system creates three output signals which are black, gray and white that are digital signals with a given logic indicating the existence of one of these colors for a particular Pixel which has an X address for a given Y position. As the scan is continued, the color profile of the total tobacco leaf being processed is obtained by a series of signals indicating the shielding effect or the light intensity of various identifiable locations or Pixels across the total surface of the leaf and also across those portions of surface 300a which are in the scanning pattern, but are not covered by a leaf.

GENERAL DATA PROCESSING

(FIGURES 4, 5, 6 AND 13)

As so far described, raw data is obtained for each Pixel or identifiable location during the scanning operation and this raw data is available at the output lines of the structure shown in FIGURE 8 or as modified by the showing in FIGURE 11. This data includes the color intensity of a given location or Pixel and the address in the X direction across the surface 300a. This information can be stored in sequence in standard READ/WRITE memory through a normal direct accessing to the memory units used in digital computers. In other words, the raw data can be stacked in sequence in the storage unit 70, shown in FIGURE 4 which corresponds to the direct access memory 220 of the control concept as illustrated in FIGURE 5. This feature of storing data relating to the color intensity of each Pixel is schematically illustrated in FIGURE 13 wherein the increment line 469 is used to index a direct accessed memory after each Pixel voltage signal is processed by the converter 420 and provided as a white, black or gray signal. To allow time for settling of the Pixel information in lines 422 (536), 432 (572) and 424 a time delay arrangement can be provided. This is schematically illustrated as a time delaying capacitor
469a. As so far described, Pixel data bits for all X Pixels
are stored in the direct access memory for each Y line or
position. Since each of the Y lines is stored, the Y address
from counter 480 shown in FIGURE 8 is not required. Indeed,
with the raw data being provided to the direct accessed
memory, the X address would not be needed since a particular
location in memory would be assigned to each of the Pixels
both in the X and Y directions. As will be explained in
accordance with the illustrated embodiment of the disclosed
system, the X address is required because not all the Pixels
are stored. In addition, the X address would be valuable
information for processing the leaf profile in most software
arrangements. Digital computer 240 uses the stored profile
data in the memory unit to locate and arithmetically convert
cut coordinates as represented by block 240a in FIGURE 13.
This function block corresponds to the processing block 82
of FIGURE 4. The cut coordinates employed in the preferred
embodiment are X1, X2 and Y which are used in the cutter
adjusting mechanism of the preferred embodiment as will be explained later. In other words, each cut position is located
by three linear coordinates which are identified for the purposes of description as X1, X2 and Y. These coordinates are
provided as eight bit digital words each of which will control
a linear moving mechanism in accordance with the magnitude of
the digital number contained in the word. The digital computer also indicates the number of the cut and the particular
cutter used together with an ACCESS signal and a PROCESS stop
signal, as schematically illustrated in the general process
chart of FIGURE 6. Essentially, the present system stores
data relating to the profile of the leaf and its defects
in a manner which can be processed to locate the

0004170

cut positions within the created profile image of the leaf. The leaf itself is not used in the location of the cutter positions. The processing of stored data to locate the cut positions is well within the general technology of the computer art when the memory has been loaded in accordance with the present system. Hereinafter, certain programming techniques will be discussed which simplify the cut location procedure used by the digital computer; however, various other arrangements could be employed for the cut location to develop cut coordinates for each cut.

<center>TRANSITION CONVERSION</center>

<center>(FIGURES 14 AND 14A)</center>

As so far described, raw data indicative of the color of each Pixel has been created. This information could be used for controlling the cutting equipment as hereinafter described by locating the wrapper cut positions based upon this raw data. However, in accordance with the preferred embodiment of the invention the raw data is not required. As long as there is raw data indicating white in the Pixels, the leaf has not been reached or a hole or white defect is being viewed. The same is true regarding a succession of gray or black raw data signals. Thus, to reduce the stored data necessary for constructing the profile or image of leaf L, the present system employs data indicative of color transitions. This transition data information can be obtained in a variety of ways between the output of surface shown in FIGURE 8 and the input to the storage memory. This involves a digital circuit interfacing the scanner with the memory unit. A digital circuit for creating transition data and usable between the scanning circuit of FIGURE 11 (FIGURE 8) and the memory units is illustrated in FIGURES 14 and 14A. Referring now to FIGURE 14, transition flip-flops 600-605 are reset when a pulse of the identified color is created. These flip-flops are set when a given color pulse is created. Consequently, the resetting of one of the flip-flops to create a logic 0 output indicates a color transition at the Pixel being processed. Upon the receipt of a reset pulse, the transition storing flip-flops 610-615 are clocked to produce a transition signal in lines 620-625 as indicated. On each Ø2 pulse, the storage flip-flops 610-615 are strobed back to the reset condition. The input lines of the circuit shown in FIGURE 14 are the output lines of the preferred embodiment circuit shown in FIGURE 11. The output lines 620-625 receive a pulse when a transition is made from one color to another color at a given Pixel during an X sweep across surface 300a. If the transition given both the "to" and "from" colors is to be used in constructing the leaf profile for processing, the signals in lines 620-625 could be used directly; however, in practice, it is only necessary to know that there is a transition to a given color such as black, gray or white. Consequently, lines 620-625 are assimilated to determine whether or not there is a transition to black, to gray or to white. Various intermediate digital circuits could be used for this purpose; however, in FIGURE 14A OR gates 630-632 having outputs 633-635, respectively, are employed. Gate 630 has an output when there is a transition to black. In a like manner, gate 631 has an output when there is a transition to gray, and gate 632 has an output when there is a transition to white. By using a digital system for determining transitions before the information is stored into the direct accessed memory, a substantially reduced number of storage locations are required to provide the necessary information for the total profile of leaf L. When transitions are being used the X address appearing on ine 472 of FIGURE 8 is required. This
determines the X posit on at which a transition is made to a
given color. If the stem is being detected, there will be a
transition to black at a given X position and then a transition
to gray after the stem has been passed. The same arrangement
is used for defects, surface variations and the edge of the leaf
to determine the profile of the leaf without using the raw data
of all Pixels. The computer would have access to the transition
information; therefore, the color of any Pixel between transitions
would be known from the stored transition information. Consequently, transition data is used to save memory capacity. The
transition circuitry shown in FIGURES 14 and 14A is in a digital
circuit between scanning circuit 200 and direct access memory 202
as schematically shown in FIGURE 5.

### FACSIMILE DATA INTERFACE AND DIRECT ACCESS STORAGE THEREOF

### (FIGURES 15-20)

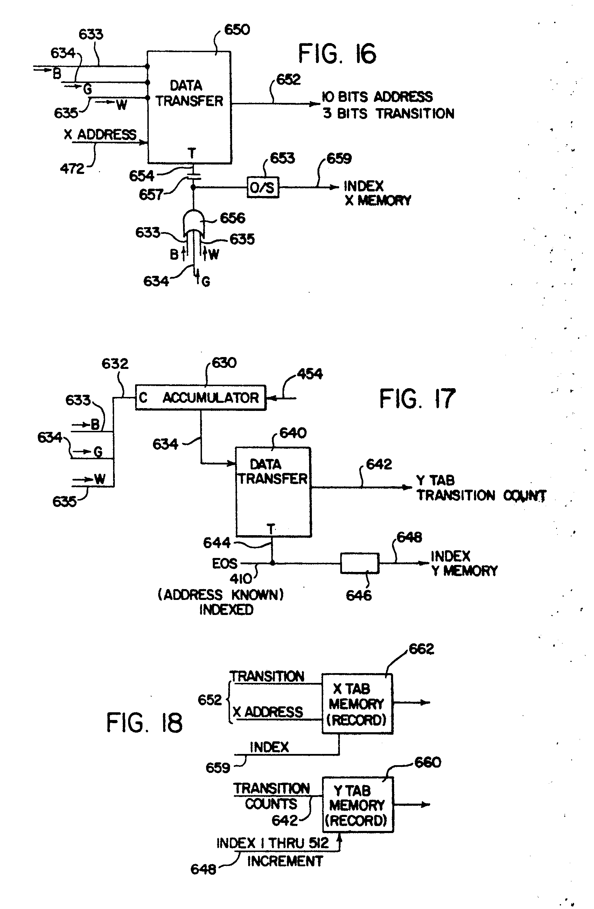

As indicated above, the raw data developed by the scanning
operation can be converted into transitions by an intermediate
digital circuitry between the output of the circuits of FIGURES
8 or 11 and the actual memory used for storing the data. Also
included in this intermediate digital circuitry for processing
transitions instead of raw data developed by the scanning process
are digital transition transferring devices of the type schematically illustrated in FIGURES 15, 16 and 17. These transfer
devices direct the transition information or data to specific
memory locations, as schematically illustrated in FIGURE 18.

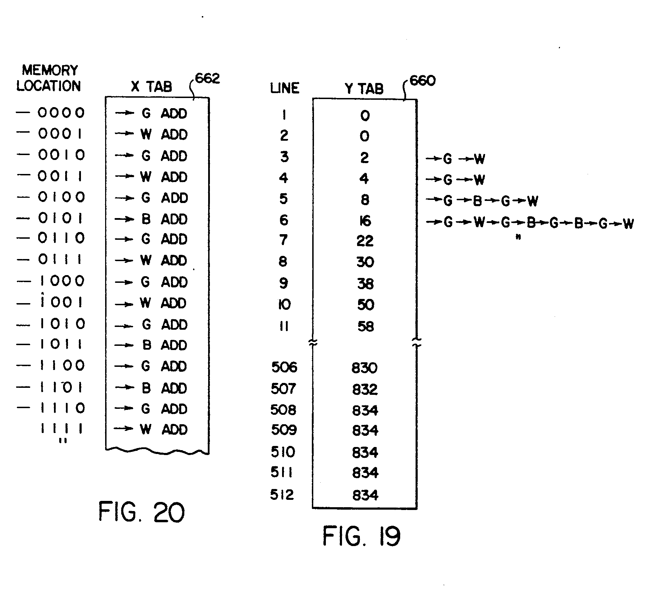

FIGURES 19 and 20 are schematic representations of the data actually stored in the memory. If the transition data is indicative
of the actual transition from one color to another color, the data

0004170

can be processed in accordance with the digital circuitry illustrated in FIGURE 15. In this circuitry, a sixteen bit accumulator 630 counts each transition pulse through a line 632. At the end of a scan, the reset line 454 resets accumulator 630. The output of the accumulator is illustrated as lines 634, which include sixteen lines to read the stages of accumulator 630. This digital data is directed to the input of a data transfer chip 640 having a sixteen bit output 642 and a data transfer terminal T activated by lines 644 which is the EOS line 410. To provide a certain time delay between the EOS pulse and the indexing of the direct accessed memory, there is provided a device, such as one shot device 646 having an output 648. The output indexes the memory to a next location into which the next accumulator data from lines 642 is stored. In operation, for each X sweep, the transitions are accumulated in accumulator 630. At the end of the sweep, the accumulated number of transitions is inserted into the memory and the memory is indexed. This continues for 512 sweeps in the X direction so that the memory has 512 sixteen bit words that are the accumulated number of transitions for each of the several Y positions of the scanning operation.

To transfer the transition data, the transitions and the X address thereof are directed to the inputs of a data transfer chip 650 having sixteen outputs 652 and a transfer terminal T controlled by line 654. OR gate 656 receives a pulse at each transition which pulse is delayed slightly by a device, such as capacitor 657, to then transfer the input data to the output lines 652 of transfer chip or device 650. An appropriate time delay 658 then allows indexing of the X memory unit by a signal in line 659. Thus, at each transition in the X direction, the address of the transition appearing in line 472 is transferred through the sixteen bit output 652 together with the type of transition. Thus, the X memory unit receives a transition and the Pixel address of the transition.

The circuitry illustrated in FIGURES 16 and 17 is the same circuitry and has basically the same numbers as the circuitry illustrated in FIGURE 15. The difference is that the transition information is received from the network illustrated in FIGURE 14A. There is only three bits of transition information or data, which data determine and record the type of transition. Accumulator 630 of FIGURE 17 still accumulates the same number of transitions; however, the data being transferred through the sixteen bit lines 652 is indicative of the transition to a particular color and not necessarily from which color the transition is being made. The circuitry shown in FIGURES 14, 14A, 16 and 17 is the preferred embodiment. This circuitry is interposed between the raw data scanning circuits of FIGURES 8 and 11 and the direct accessing memory units. This is schematically illustrated in FIGURE 18 wherein the direct access memory unit for the Y information is called the "Y TAB" memory 660. The accumulated number of transitions at each of the Y positions is recorded and stored in sequence in this unit. In a like manner, the X information including the type of transition and the Pixel address for the transition is directed to the "X TAB" memory 662. This memory is indexed at each transition. Thus, the Y tab includes the accumulated number of transitions for each of the 512 different Y lines being scanned by the scanner mechanism 200. The X TAB memory location includes the Pixel at which a transition is made by an X address and the type of transition. By using both the information and the X tab and Y tab direct access memory units, a profile of the leaf is stored for processing by an appropriate software arrangement for locating profile 30 within locations of the
leaf L where there are no defects. The general outline of
the memory data in the Y tab and X tab storage units or
memories are schematically set forth in FIGURES 19, 20,
respectively.

PREFERRED DATA PROCESSING CONCEPTS

(FIGURES 21-26)

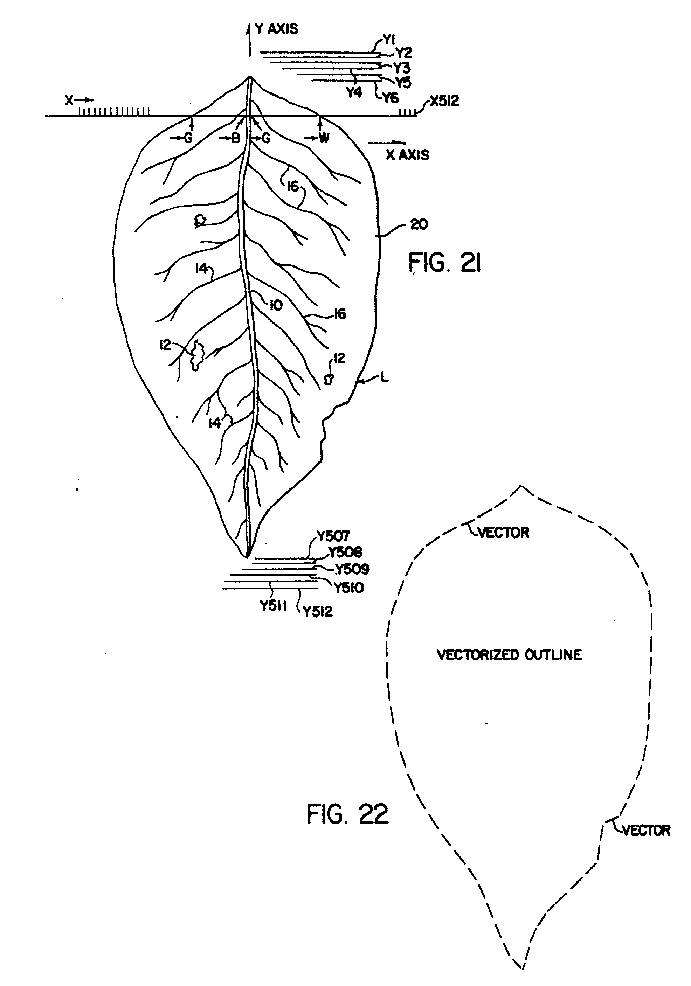

As previously explained, the raw data or transition data,
in the preferred system, is transferred to the memory 200 for
use by the digital computer 240. The units 600, 602 of the
preferred example correspond to memory 200 of the control
diagram of FIGURE 5. The computer then creates the cut locations and computes the cut coordinates for the various wrapper
cuts to be made from leaf L. This can be done by a variety of
software packages; however, in accordance with the present
system certain concepts have been developed for use in reducing the computer time and the software complexity. These
basic concepts are set forth in FIGURES 21-26. Referring now
to FIGURE 21, leaf L is shown with the X and Y coordinates of
the scanning grid. In the single illustrated line X, it is
noted that there is a transition to gray at the leaf edge, a
transition to black at stem 10, a transition to gray after
the stem and then a transition to white at the opposite leaf
edge. This type of pattern continues through the total X
sweep. Other transitions are also experienced. After the leaf
has been passed, the subsequent X sweeps do not provide
further transitions. At the first X sweep, there is usually
no transition. Thus, the Y tab storage as shown in FIGURE 19
can indicate the length of the leaf. Also, the Y tab can
locate the X transitions for a given Y scan by pointing to a
required X tab location. For instance, if the sixth Y
line is being read, the Y tab indicates that the X sweep is
between the 8th and 16th transition of the X tab.
The X tab is scanned between the 8th transition and the 16th transition to give the X sweep for the sixth Y line.
If the 11th Y line is to be analyzed, the X tab
between the 50th and 58th transition provides the X
sweep profile.    This concept provides an easy access
to the X sweep profile for processing by the computer
by using transitions and accumulated transitions for
locating information in the X tab.    There is no need
for a Y tab address although it is available, since
the Y tabs are serially stored data between 0-512.
By taking this information, in accordance with the
preferred data processing system, a vector of the
leaf outline shown in Figure 22 is created by
analytical geometry.    This outlining vector concept
provides two X coordinates at a given Y line.    The
first X coordinate is a first transition from white
and the second X coordinate is a last transition to
white.    Thereafter, the Y line is indexed a set
number, such as 20-40 lines.    The next Y line gives
two additional outline X coordinates.    These X
coordinates are then noted and a mathematical vector
is created in the computer for comparing cut profile
30 (in vector form) with the vectorized leaf outline
to prevent interference with the leaf edge.

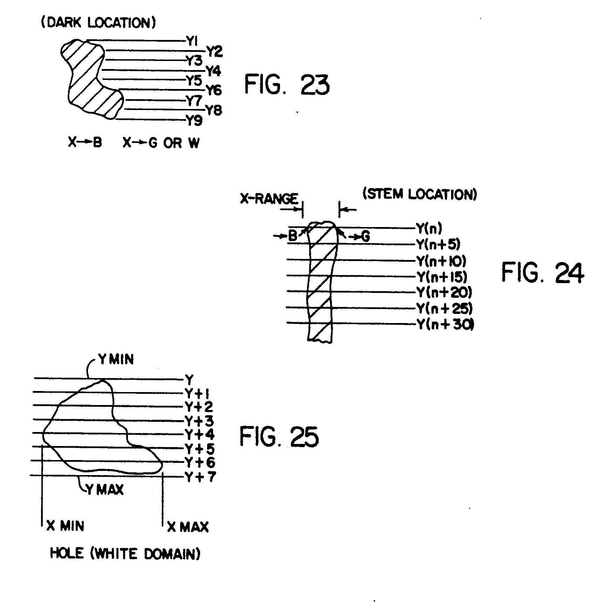

A dark area is located and recorded as shown
in Figure 23.    Each of the Y lines is scanned for a
transition to black and then for a transition to gray
or white.    This indicates a black area on the leaf at
each of the Y lines.    To locate holes in the leaf,
each of the Y lines is scanned for a transition to
white and then to gray or black.    For each hole, a
maximum and minimum X and a maximum and minimum Y is
recorded in the memory profile to outline a hole area
or domain which is rectangular and is to be avoided
by a cut.    A stem is located by noting a series of
transitions to black and then to gray along the stem.
If these transitions are aligned in the X direction
for a successive number of Y positions, such as 5-10 lines, it is noted that the stem has been located for storing in a memory unit of the computer. If the transitions do not align in the X direction over successive Y lines, the transitions are not the stem 10 and are known to be either the diagonal veins or dark locations previously recorded. By vectorizing the outline of the leaf, constructing a rectangle or domain around the holes, noting the black or dark locations and locating the stem, there is sufficient processed information to locate the coordinates of a cut for a wrapper to avoid the stem, dark areas, white areas and holes, as well as the outline of the leaf. FIGURES 22-25 disclose concepts used to facilitate the creation of a total leaf facsimile in X and Y coordinates and vectors therebetween for location of proper cut positions. These are novel processing concepts and are not mathematical in nature.

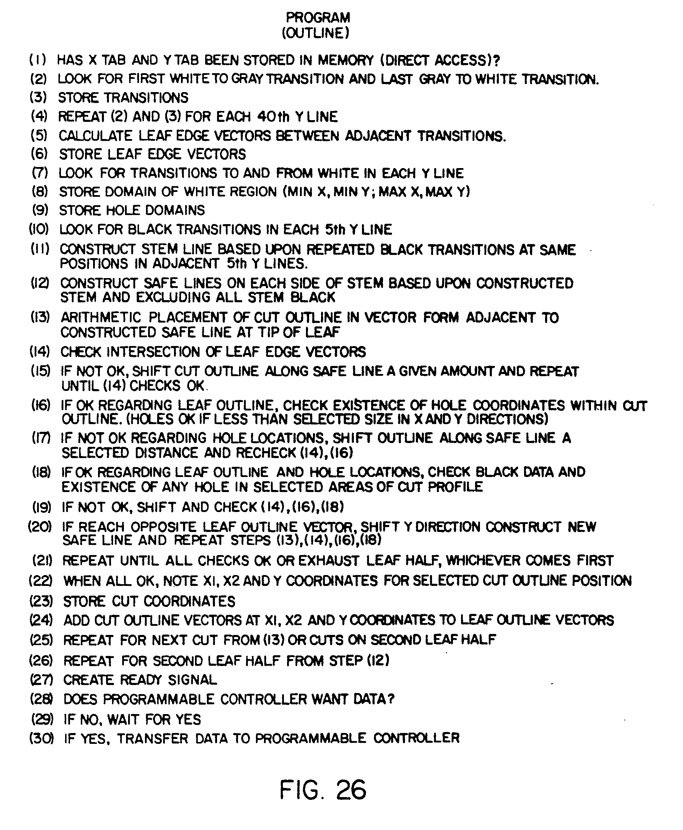

In FIGURE 26, the general programming approach to the preferred embodiment of the invention is illustrated. Basically these program steps which could be set forth in block diagrams are self-explanatory in nature when considering FIGURES 19-25 and can be accomplished by various software routines and techniques. The concepts of FIGURES 19-25 are novel procedures which can be performed by software and used in the general program of FIGURE 26.

In the general program outline, after the X and Y tab have been stored in memory, steps (2)-(6) create the vector outline of the leaf as shown in FIGURE 22. Steps (7)-(9) provide the hole domain as shown in FIGURE 25. Steps (10) and (11) construct the stem for the leaf which is to be avoided in the cut position. By an optimizing subroutine, a safe line is constructed adjacent both sides of the stem as shown in step (12) which safe line is to be avoided in positioning a cut profile 30. In step (13), the vectorized profile 30 is mathematically positioned at one end of the leaf and adjacent the stem safe line. In step (15), if there is an intersection of the edge vector as tested in step (14), the cut profile is shifted along the safe line a given amount and step (14) is repeated. If the outline is avoided as indicated in step (16), the existence of holes or other unwanted defects is determined. If there are holes or other defects not wanted in the profile 30, it is then shifted in the Y direction along the safe line adjacent the stem as in step (17) until a position is located wherein there is no hole location or leaf outline intersection. At that time, as indicated in step (18), the black area and any minute holes in the tuck or head are tested as indicated in step (18). As indicated in step (19), if the tuck or head has black areas or other small defects the shifting process continues until the cut profile vectors reach the opposite leaf vector outline or a cut location has been determined. If the outline has been reached without a cut location, a new safe line is constructed as indicated in step (20) which is offset (in the X direction) and spaced from the previous safe line. The process is repeated until whole leaf half has been exhausted as indicated in step (21) or a cut location has been obtained. If the cut location has been obtained and meets the parameters previously discussed, the X and Y position of a point on the profile 30 and the angle of this line with respect to the Y axis is recorded and the necessary X1, X2 and Y coordinates are calculated and stored for subsequent outputting to the programmable controller to use for subsequent cutting. The angle $\emptyset$ occurs because the stem may not be aligned in a Y line which will give an angled safe line. This angle is small since the leaf L is aligned as best shown in FIGURE 21 with the stem as vertical as practical. The type of

0004170

coordinates which are required are correlated with the type of movement mechanism being used for the cutting device so that the mathematics for creating coordinates will create an output that controls the position of the cutting in accordance with the selected cut position originally identified as X, Y and $\emptyset$. In other words, the X1, X2 and Y coordinates are calculated from the X, Y and $\emptyset$ coordinates to correspond with the exact type of cutting mechanism used to adjust the leaf supporting surface or cutting surface with respect to the cutter as will be explained later. This mathematical relationship is easily programmed into the computer. This function is steps (22) and (23). After a cut position has been located, as indicated in step (24), the vectors used in the profile 30 are added to the outline vectors of the leaf to change the profile of the leaf vectors by excluding from future consideration the previously selected cutting position. Thereafter, as indicated by step (25), the process from step (13) is repeated to locate a next cut or cuts on the half of the leaf being so far processed. After all the cuts have been located on one half of the leaf, the steps commencing at step (12) are repeated for the second half of the leaf. Thereafter, the program has been completed and the computer will create a ready or access signal as indicated by step (27). This can be an output flip-flop or other arrangement to indicate to the programmable controller that the cut coordinates have been determined for a particular leaf being scanned and processed. As indicated in steps (28)-(30), the computer then waits until the programmable controller 210 indicates that it is ready to receive the cut coordinates, the cutter and cut numbers from the digital computer for use in subsequently cutting cigar wrappers from leaf L. Of course, other programming concepts could be used in practicing the present system, but the information illustrated in FIGURES 18-26 is preferred and involves certain novel concepts which reduce the software involved; decrease the cycle time for the total software program and reduce the chances of any error in the location of the cut positions for various cigar wrappers from a given natural tobacco leaf L. Basically, the outputted coordinates X1, X2 and Y are binary words that are transferred from the digital computer to the programmable controller for controlling the cut location determined by the computer. The digital information from the computer is then used directly for the location of the cut positions in a manner to be described later.

<div align="center">

POST SCANNING PROCESSING
MACHINE COMPONENTS

(FIGURES 27-39)

</div>

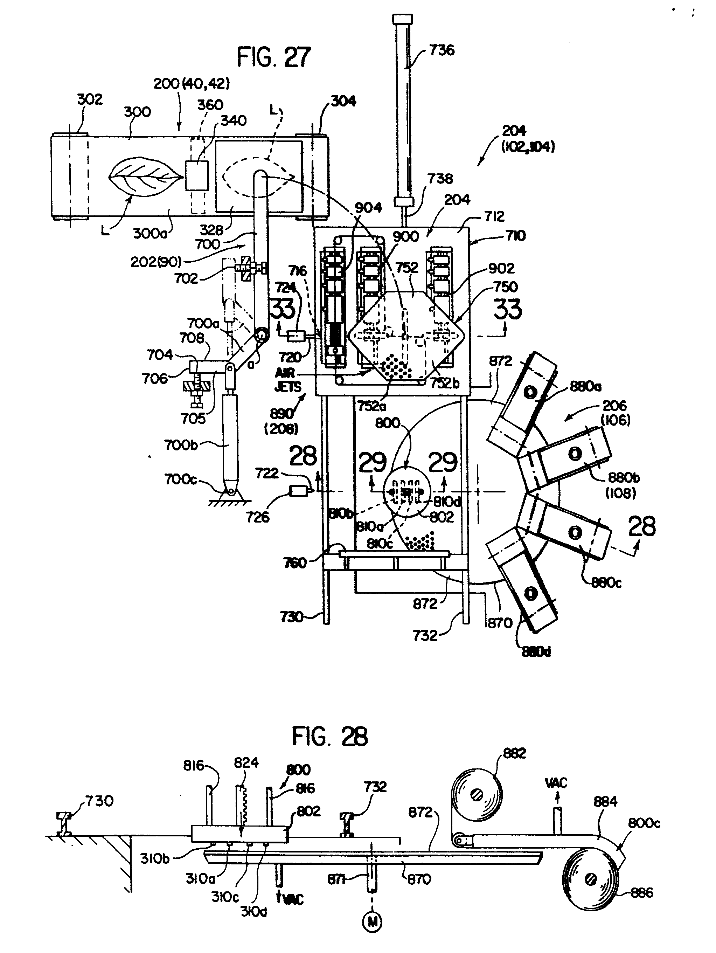

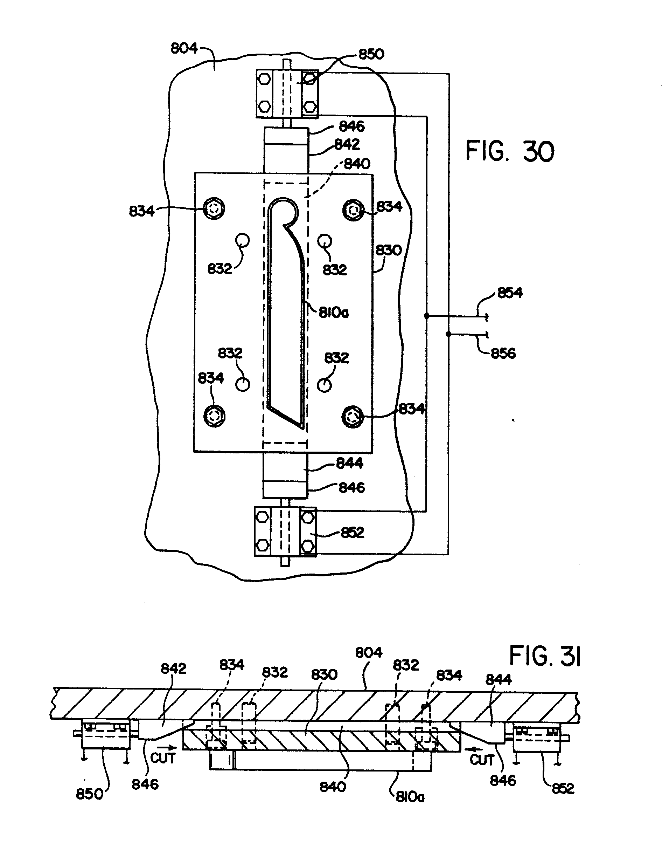

Referring now again to FIGURE 5, the apparatus for performing the scanning and cutting process to remove cigar wrappers from leaf L includes several machine components which are generally labeled in this control concept diagram and are shown in detail in FIGURES 27-39. Essentially, the programmable controller 210 receives coordinates X1, X2 and Y for each of several cuts to be taken from the leaf together with the proper cutter and the sequence of cuts to be made. This information is received in digital form with the three coordinates in the preferred embodiment being binary representations of translation or linear movement. Programmable controller 210 uses the binary coordinates and the digital information to control the various mechanisms including the scanning mechanism previously described to process the leaf so that the computer itself can be used primarily for determining the cut positions based upon direct stored memory information indicative of the profile or image of the leaf after scanning by mechanism 200 The machine cycles controlled by the programmable controller are in accordance with common machine control techniques; therefore, the mechanisms will be described in accordance with their structure and functions.

Scanning mechanism 200 has been previously described in connection with the structure of FIGURE 7; therefore, the detail of mechanism 200 is not to be repeated. Details of the other machine components will hereinafter be set forth in an embodiment of the invention to perform the desired functions; however, it is appreciated that other embodiments could be used. In some instances, the disclosed embodiments illustrate novel features which were specifically developed for the present system and which will be the subject of certain claims on the inventive aspects of the present application.

## LEAF TRANSFER MECHANISM

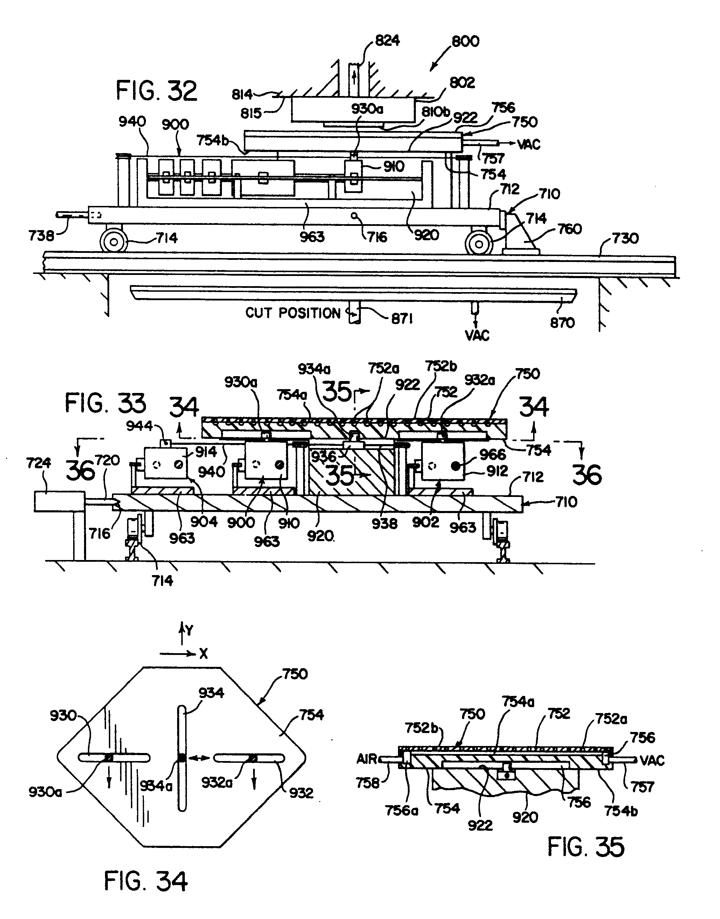

As shown in FIGURE 27, the leaf is transferred from surface 300a of belt 300 by a somewhat standard vacuum type transferring arrangement which includes an arm 700 supporting a head 328 which includes a lower perforated surface generally parallel to the upwardly facing surface 300a of belt 300 in the transfer area of the scanning arrangement as shown in FIGURE 7. For the purposes of simplicity in FIGURE 7, arm 700 which directs either air or vacuum to head 328 is shown to be generally horizontal of belt 300; however, as shown in FIGURE 27 the arm in the transfer position is essentially transverse of the belt 300. Head 328 is shifted by arm 700 between a first leaf receiving position and a second leaf releasing position. Arm 700, in the illustrated embodiment, is turned by lever 700a about axis a by a cylinder 700b mounted on fixed trunnion 700c. The first leaf receiving position of arm 700 and head 328 is located by an adjustable stop 704 coacting with surface 705 of extension 706. A second adjustable stop 702 coacts with surface 708 of extension 706 to locate head 328 in the leaf releasing position. Limit switches at these stops will indicate when the transfer head 328 carried by arm 700 is in either of the two positions. Appropriate valving will direct either air or vacuum to the perforated under surface of head 328 for lifting a scanned leaf from belt 300 or depositing a leaf onto the cutting table. The belt must be in a known position with respect to the scanning operation when the leaf is removed from the belt. In this manner, the position of the leaf on the transfer head 328 is correlated and oriented to the Pixels scanned during the scanning operation. This can be done by stopping belt 300 at a fixed number of encoder pulses in line 312 after the stop scan signal in line 454 of FIGURE 8. Other systems could be developed for assuring that the leaf is captured on head 328 in direct correlation to the X and Y scanning lines of the scanning operation. Irrespective of the technique used, leaf L is picked up by transfer head 328 in a fixed position with respect to the prior scanning operation which maintains the positional orientation with respect to the prior scanning of the leaf. Adjustable stop 704 allows small adjustments in the pick up position. Thereafter arm 700 is shifted by cylinder 700b against stop 702 which transfers the leaf held against the lower surface of head 328 to a fixed, known position with respect to platen 710 having an upper surface 712. Small adjustments can be made by stop 704. Leaf L is deposited onto platen 710 in a known position with respect to the prior scanning operation. Platen 710 includes lower guide wheels 714 and a side locator hole 716 into which a pin 720 or 722 is protruded by an operator, such as a solenoid 724, 726, respectively. In the leaf transfer position of platen 710, as shown in FIGURE 27, pin 720 is in
hole 716 so that the platen is in a fixed, known position.
Transversely extending support rails 730, 732 allow movement
of platen 710 by appropriate means schematically represented
as cylinder 736 having a movable rod 738. Carried upon the upper
portion of platen 710 is a cutting table 750 onto which the
leaf is transferred when arm 700 is against stop 702. Cutting
table 750 is in the oriented fixed position as shown in FIGURE
27 for receiving the leaf. By coordination of the table 750 and
the pick-up position of leaf L from belt 300, the leaf is deposited
by transfer head 328 onto table 750 in a coordinated, oriented
position with respect to its previous scanned position. The
table 750 is supported on platen 710 which is held in the leaf
receiving position by pin 720 entering guide slot 716. The
scanned leaf which produced the desired profile in a stored memory
unit is transferred onto the oriented table 750 by applying a
vacuum to the cutting table and pressurizing or evacuating the
head 328. This is a known transfer arrangement for tobacco in
the cigar making industry. As best shown in FIGURES 32-35, cutting
table 750 includes an upper nylon plate 752 having a plurality of
parallel arranged openings 752a which have a diameter of approximately 0.04 inches. This plate, except for holes 752a, is a
cutting board produced by Boston Cutting Die Company and is formed
from hard nylon with a trademark WOGULON. Upper plate 752 has an
upper cutting surface 752b. A second hard nylon plate 754 is formed
with a plurality of generally parallel grooves 754a that correspond
with openings 752a and have a thickness of about 3/16 of an inch.
Inbetween the grooves the upper and lower plates 752, 754 are secured together for cutting rigidity. Grooves 754a do not extend to
the periphery of cutting table 750 and they form a plenum chamber communicated with the openings 752a to allow a vacuum from manifold 756 to be applied to all grooves 754a and thus to the openings 752a at surface 752b. Another manifold 756a may be positioned at the opposite end of grooves 754a. In the illustrated embodiment shown in FIGURE 35 a vacuum is applied to manifold 756 so that a vacuum can be directed to the upper surface 752b of cutting table 750 for clamping a transferred leaf onto the upper cutting surface for performance of the cutting operation. To remove a spent leaf from the surface 752b after the cuts have been made air pressure can be directed to grooves 754a by line 758 communicated with manifold 756a. Of course, a single manifold could be used at one end of the grooves with either vacuum or air being applied selectively to the same manifold. The manifolds are positioned generally outside the cutting area of cutting board or table 750 so that the manifolds do not adversely affect the rigidity of the board or table for the cutting operation. By providing the parallel grooves to direct pressure to the surface 752b of the cutting board or table 750, the cutting table is essentially a rigid, hard nylon cutting structure. As can be seen, vacuum holds the leaf onto the cutting board in the position to which it is transferred by transfer head 328. This position is oriented and coordinated with the scanning operation so that the leaf is in the desired position for adjustment with respect to a cutting mechanism to be hereinafter explained.

## CUTTING MECHANISM

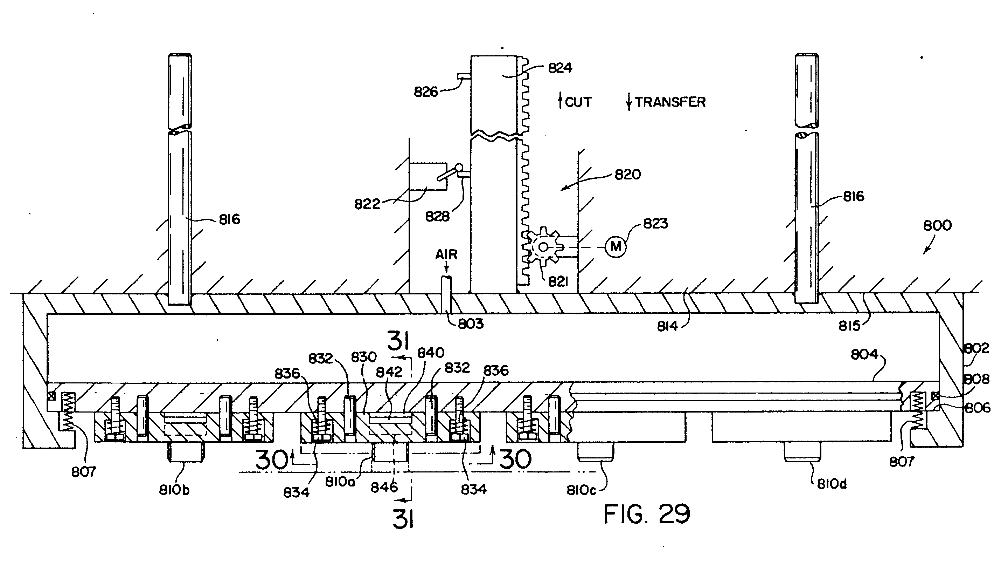

Before describing the system for adjusting table 750 to the desired position for cutting, it is desirable to understand the preferred embodiment of the cutting mechanism 800. This mechanism is best shown in FIGURES 29-31 and includes a pancake cylinder 802 having a pneumatic inlet 803 and a piston 804 movable against a lower abutment or shoulder 806 and spring biased away from the abutment by a plurality of circumferentially spaced machine springs 807. Of course, an appropriate seal 808 is employed to seal the cylinder and piston. As illustrated, four separate cookie cutter type dies 810a-d are provided on piston 804. In practice, it is conceivable that only a left hand and right hand cutting die will be used. By showing four separate cutting dies, the system can cut two separate sized wrappers from each half of the leaf by an appropriate signal from the computer to the controller. This is useful when a large wrapper is being cut from the natural tobacco leaf and it is found that a smaller wrapper could be cut from available non-defected areas instead of one or more larger wrappers. The use of more cutting dies would increase the productivity; however, it is not necessary for the general understanding of the system. Basically, the computer commands the programmable controller 210 where to cut and which cutter 810a-d to use. Normally, the cutters 810a and 810c would be used to produce wrappers having the same general shape, but cut from opposite sides of the leaf so that they would be opposite profiled wrappers for use in different cigar making machines. The cutters themselves are fixed in position and the table 750 adjusts the tobacco leaf carried thereon to the proper cutting position. In the illustrated system, cylinder 802 is mounted on a fixed machine frame 814 having a lower abutment surface 815. A plurality of slidable guide pins 816 allow reciprocal movement of cylinder 802. A mechanism 820 is provided for shifting cylinder 802 between the upper cutting position with the cylinder against surface 815 and a lower position for transferring the cut wrapper to a subsequent apparatus for storage and use. Mechanism 820 could take a variety of forms; however, in the illustrated embodiment this mechanism includes a limit switch 822 to control the vertical position of cylinder 802. A pinion 821 driven by an appropriate motor 823 coacts with rack 824 secured on cylinder 802 to drive the cylinder between the upper cutting position and lower transfer position. The basic location of these two vertical positions is generally controlled by an appropriate mechanism, such as cams 826, 828 coacting with limit switch 822. During the cutting operation, cylinder 802 is abutting against surface 815 to rigidify the cylinder. During the wrapper transfer operation, rack 824 shifts cylinder 802 downwardly.

Cutters 810a-810d are located on piston 804 and are essentially the same in structure, except the size and/or shape of the cutters are somewhat different to create wrappers having the desired shape. It is appreciated that any number of cutters could be mounted on the piston and can be selectable for the actual cutting operation. Since each of the cutters, except for shape, is the same, only cutter 810a will be described in detail. This description will apply equally to the other cutters mounted on the piston. Indeed, only one cutter may be provided in some instances. Cutter 810a is fixed to a cutter block or support block 830 slidably received upon a plurality of dowl pins 832. Appropriately spaced machine bolts 834 include machine springs 836 which hold block 830 rigidly against the undersurface of piston 804. A slot 840 extends across the back surface of block 830 in a position generally parallel to the wrapper cutter 810a. Opposed cams 842, 844 having a cut supporting upper surface 846 are movable by solenoids 850, 852 under the control of a signal received by lines 854, 856. If a particular cutter is selected to make the cut being processed by the mechanism as shown in FIGURE 27, solenoids 850, 852 shift cams 842, 844 inwardly until surfaces 846 project block 830 outwardly on dowl pins 832. In this fashion, surfaces 846 support cutter 810a in a downwardly extended active position with respect to all other cutters which remain in a retracted inactive position. Thus, only cutter 810a will cut when piston 804 is forced downwardly by pressure from inlet 803 against the action of machine springs 807. The particular signal indicating the cutter to be used controls the signal within lines 854, 856 to select any one of the cutters supported on piston 804. When piston 804 is forced downwardly against table 750, the extended cutter cuts according to the adjusted position of the table with respect to that activated cutter. As the piston 804 is retracted by exhausting air from line 803, the cigar wrapper cut by cutter 810a is held within the cutter and pulled from the surface of leaf L. To assist in this action, a vacuum, from a source not shown, may be directed to the inside or the interior of the cutter. With the wrapper cut and within the cutter, cylinder 802 can be shifted downwardly after platen 710 is retracted from the cutting position. The wrapper is now ready to be transferred for subsequent processing.

TRANSFER OF CUT WRAPPER

Referring now to FIGURES 27, 28 and 32, a cut wrapper is transferred to the vacuum table 870 indexable about center shaft 871. The upper surface 872 of the table is perforated at least in selected areas. A plenum chamber below surface 872 can apply a vacuum to the surface in accordance with standard practice. Of course, certain areas of the plenum could be formed to create surface vacuum, air pressure or atmospheric pressure to assist in wrapper transfer. When cylinder 802 is moved downwardly by rack 824, cutter 810a having a cut wrapper therein is positioned onto surface 872. Thereafter, air pressure can be applied to the cutter profile or the vacuum itself in the table 870 can draw
the cut wrapper from the cutter onto surface 872. Cylinder 802
is then retracted upwardly for the next cutting cycle and table
870 is rotated or indexed to one of the standard wrapper storage
bobbins 880a-880d. Each of the bobbins receives a cut wrapper
from only one of the cutters 810a-810d. The standard wrapper
storage bobbins 880a-880d each includes a porous belt 882 on a
storage reel, a vacuum transfer and holding housing 884 and a
storage bobbin 886, as shown in FIGURE 28. After a wrapper cut
has been made, the wrapper is deposited onto table 870 which
is indexed to the desired position where a vacuum is applied to
belt 882 at least adjacent the inlet end of housing 884. The
cut wrapper is transferred to belt 882 and held onto the belt
until it is reeled onto bobbin 886. During the indexing of
table 870 and storage of a cut wrapper the next cutting operation
is being performed by mechanism 204.

This wrapper removal procedure follows each cut and is repeated until all cuts are made in leaf L. The wrappers are all
stored on the appropriate bobbins 880a-880d. Platen 710 carries
table or cutting board 750 back to the leaf receiving position
shown in FIGURE 27 where the table 750 is pressurized by line 758
and air jets 890 blow the cut leaf off the table into an appropriate
repository. This removing mechanism using jets 890 is schematically
illustrated as block 208 in FIGURE 5.

### CUT LOCATING MECHANISM

After the cut positions have been located and identified as
three digital words (X1, X2, Y), these words or coordinates are
used to locate the cut positions of cutting board or table 750.
To perform the movement of table 750 to the various cut positions
platen 710 carries table 750 to the position shown in FIGURE 32.

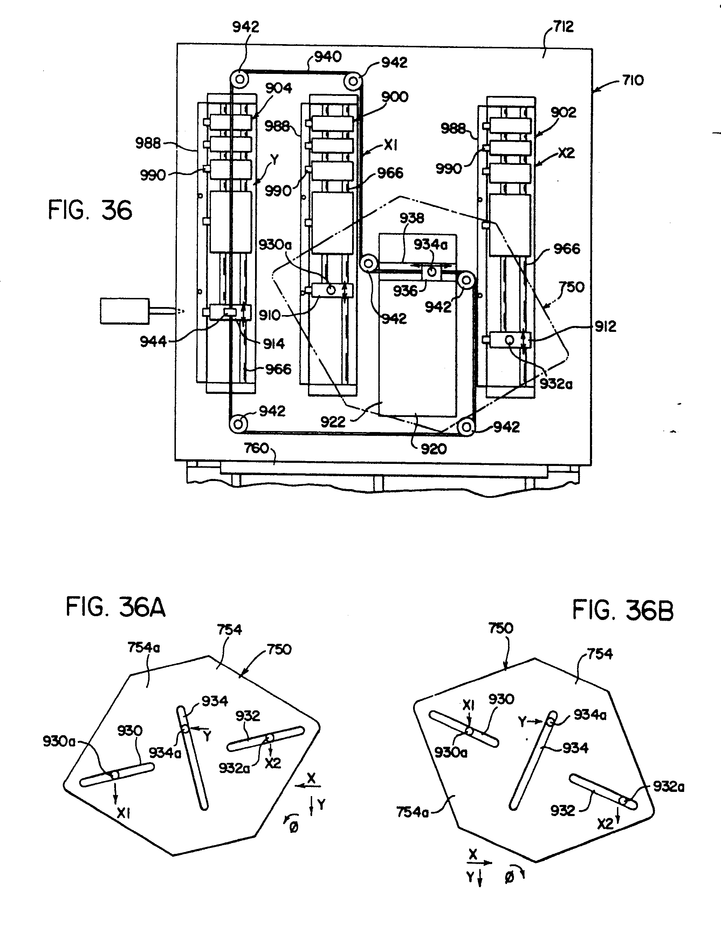

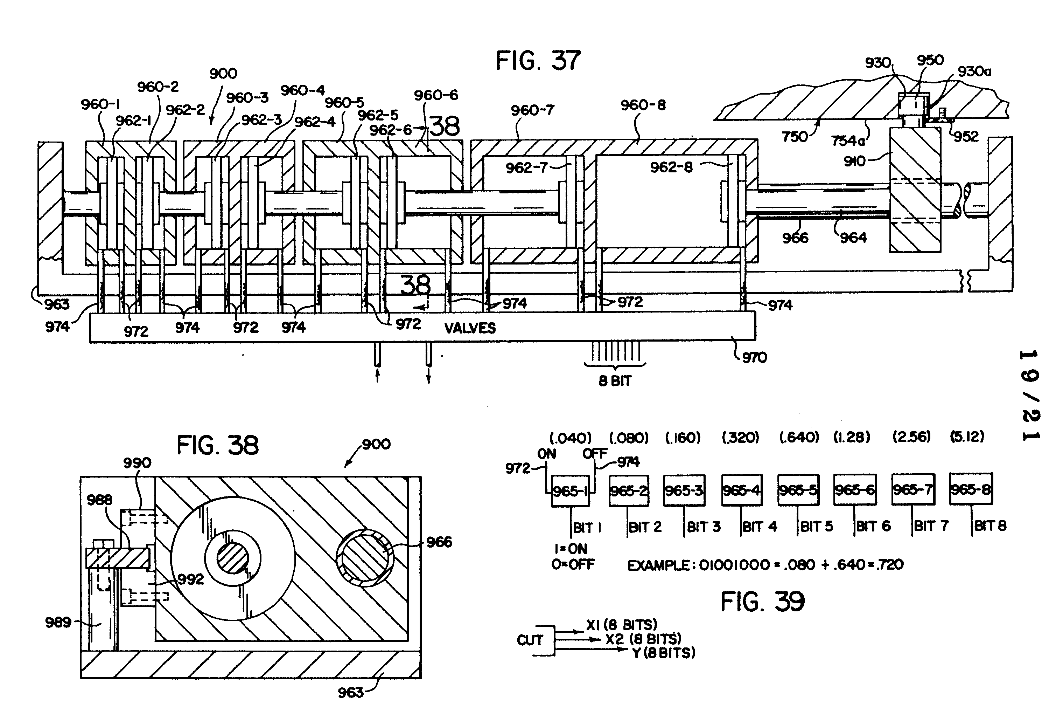

During movement of the platen to the cutting position, or after the movement has been completed, table 750 is shifted, rotated and otherwise moved to align leaf L directly below the selected cutter, which for illustrative purposes has been indicated to be wrapper cutter 810a, in the cut position identified by coordinates X1, X2 and Y. This shifting of table 750 is done by converting the three digital words (X1, X2, Y) corresponding to the proper cut position into three coplanar translation movements of the table 750 which duplicate the X, Y and $\emptyset$ cut position previously described. In accordance with a novel aspect of the present system, the three linear movements, which could be coordinates X, Y and $\emptyset$ as shown in FIGURES 36A, 36B, are obtained by linear translation distances determined by the binary number of the three separate words X1, X2 and Y. Of course, according to the type of moving mechanism used for table 750, the translation magnitude words will vary. The translation distances cause table 750 to move so that the located X, Y, $\emptyset$ position of leaf L is aligned with the selected cutter. In accordance with this aspect of the system, no rotary movement is required for table 750. The angular movement corresponding to $\emptyset$ is obtained by differential linear translation movements of two linear motors 900 and 902 fixed on surface 712 of platen 710. The details of these motors will be explained in connection with FIGURES 37-39. The motors 900, 902 and 904 each carry a movable element 910, 912, 914, respectively, which are translated a distance controlled by the binary magnitude of digital words X1, X2 and Y, respectively. These words are directly correlated with the movement of elements 910-914 to locate table 750 in the cut position as determined by the scanning and selecting process previously described. The location of table 750, in turn orients leaf L carried in a known relation on surface 752b.

Of course, various systems could be used for movement of table 750, some of which would include a rotary action. It has been found that by using the translation action developed in accordance with the present system, the difficulties encountered in rotating table 750 through an angle determined by a cut coordinate is avoided. As previously discussed, the cut position can be at an angle with the Y axis since the stem of the leaf may be at an angle or the leaf may be positioned on belt 300 at a slight angle. For that reason, the preferred system requires a certain amount of angular movement which is obtained by differential translation of the two sides of table 750 by motors 900, 902.

The lower surface 754b of the lower nylon plate 754 forming table 750 rides along and is supported by an anvil 920 having an upper table support surface 922 which is parallel to lower surface 754a. Anvil 920 supports table 750 during the shifting action to locate the various cut positions. To move table 750 into the cutting positions there are provided two generally parallel slots 930, 932, as best shown in FIGURES 34, 36A and 36B. These slots are milled or otherwise provided in the lower nylon plate 754 of table 750 and are generally parallel in relationship and extend in the X direction of table 750. Between these two slots there is provided a generally orthogonal slot 934 which is at an oblique angle with slots 930, 932 which angle, in practice, is 90° so that it is aligned with the Y axis of table 750. The X and Y axes of table 750 define a grid corresponding to the scanned grid of the leaf. Of course, the grids are not marked on the scan surface or table since they are correlated by the transfer of leaf L. Pins 930a, 932a, and 934a are slidably received within slots 930, 932, and 934, respectively, so that movement of the pins in a direction generally transverse to the slots will orient table 750 for proper positioning of leaf L at the proper cutting position with respect to the selected cutter, illustrated as cutter 810a. Pins 930a, 932a move in straight lines on platen 710 which lines are parallel to the Y direction of table 750 when the table is in the leaf receiving position shown in FIGURES 27 and 34. Pin 934a is movable in a straight line on platen 710, which straight line is parallel to the X direction of table 750 when the table is in the leaf receiving position shown in FIGURES 27 and 34. In the illustrated embodiment, as best shown in FIGURE 34, slots 930, 932 are aligned and on opposite sides of slot 934 and pins 930a, 932a and 934a are all aligned in a direction corresponding to the X direction of table 750 when the table 750 is in its leaf receiving position shown in FIGURES 27 and 34. Pins 930a, 932a are directly supported upon the movable elements 910, 912, respectively, of motors 900, 902, respectively. Consequently, reciprocation of elements 910, 912 causes reciprocation of pins 930a, 932a to reciprocate table 750 in the directions indicated by the arrows in FIGURE 34. Pin 934a is carried by a block 936 slidably received within slot 938 of anvil 920. A drive cable 940 with a series of pulleys 942 connect block 936 drivingly with an anchor block 944 supported on element 914 which is reciprocated by the third translating motor 904. Slot 938 is orthogonal to the rest position of slot 934 and parallel to the direction of slots 930, 932. Thus, translation of element 914 causes pin 934a to move in the direction indicated in FIGURE 34 a distance equal to the translation distance of element 914.

As three digital words are received, they indicate in binary language the amount of translation of the elements 910, 912, 914. The words actually provide the translated position of elements 910-914 for the cut position. If the motors 900-904 start from a zero position, the actual word gives the amount of translation. If the elements are in an intermediate position the words indicate the corrective amount of translation. Motors 900-904 may be at a prior cut position and they need not retract to the zero position to go to the next cut position. In practice, table 750 is in the zero positions of X1, X2 and the middle position of Y when in the cut receiving position shown in FIGURES 27 and 34. The binary words X1, X2 and Y are converted into movement of table 750 by the coaction between slots 930-934 and pins 930a-934a. In FIGURE 34, table 750 is in the rest or leaf receiving position, which is the position used in accepting a leaf when platen 710 is in the leaf receiving position shown in FIGURE 27. After the leaf has been received and vacuum has been applied through line 757 of table 750, the leaf is held in place and the programmable controller receives cut coordinates X1, X2 and Y which control the translation position of elements 910-914 controlled by motors 900-904, respectively. In this manner, the table 750 carrying the leaf is adjusted with respect to the cutter 810a, which cutter is then forced downwardly against table 750. When this happens, the upper surface 922 of anvil 920 supports the table to create a reactive force against the table to facilitate the cutting action. Thus, the movement of the cutting board or table 750 into the proper cut position is by the movement of three pins which are translated, but do not support the table in a vertical direction. The actual cutting force is against anvil 920 with upper portion 752 of table 750 being the actual cutting member. In this manner, table 750 has a low weight and develops low inertia forces. Pins 930a-934a need not have a heavy construction to withstand the subsequent cutting forces. Thus, the pins develop low inertia forces. In practice, a head is provided on the moving pins 930a,932a to coact with a retaining structure adjacent slots 930,932 to hold table 750 in the horizontal position as it is shifted. As previously mentioned, after the cut has been made the wrapper is removed by the cutter and platen 710 moves to a position clearing cylinder 802 so that the cylinder can be moved downwardly to transfer the cut wrapper onto vacuum table 870 for subsequent indexing to one of the wrapper bobbins 880a-880d. When the cut is made by cutter 810a, table 870 is indexed to wrapper storage mechanism 880a where the cut wrapper is transferred to storage element or bobbin 886 for subsequent loading into a cigar machine in accordance with standard cigar making practice. As shown in FIGURE 37, the top of the pins 930a,932a can include a head 950 held within the respective slots by an appropriate plate 952. Other arrangements, such as a T-slot and head, could be used to hold table 750 onto the moving pins for movement therewith. The actual cutting action takes place against the upper surface 922 of anvil 920.

FIGURE 36A shows an example where table 750 has been shifted to a cut position with X1 greater than X2 and Y shifted to the left, as viewed from surface 754a. Another example is shown in FIGURE 36B with X2 greater than X1 and Y shifted to the right as viewed from the under surface 754a.

A variety of structures could be used to translate elements 910-914 in a manner controlled by digital signals; however, one mechanism for accomplishing the conversion between digital coordinates in binary words and translation of the movement elements is a binary motor concept. This concept is used in motors 900-904. Since each of these motors is essentially the same, only motor 900 will be described in detail and this description will apply equally to binary motors 902 and 904. As shown in FIGURES 37-38, internal double acting pistons 96: -1 to 962-8. These cylinders are connected together so that an eight bit binary word used to control the pressure in each of the cylinders will translate element 910 a distance corresponding to the numerical value of the binary number. Of course, the binary number could include various number of bits. A variety of structures could be used for interconnecting the pistons and cylinders; however, in the illustrated embodiment the cylinders are connected together in the following sets: 960-1 and 960-2, 960-3 and 960-4, 960-5 and 960-6, and 960-7 and 960-8. Piston 962-1 is fixed at one end of motor frame 963. The pistons are interconnected in the following sets: 962-2 and 962-3, 962-4 and 962-5; 962-6 and 962-7. Piston 962-8 is connected to the movable element 910 by shaft 964. Successive pistons have strokes which vary in binary fashion, i.e. vary as a factor of 2. In the illustrated embodiment as set forth graphically in FIGURE 39, the first cylinder has a stroke of 0.04 inches. Each of the successive cylinders has strokes which are factors of 2 of this stroke. A support and bearing rod 966 extends the complete length of motor frame 963 and passes through the respective cylinders as shown in cross-section in FIGURE 38. This rod allows translation of the various cylinders in a direction parallel to the stroke of the pistons within the cylinders. To support the other side of the cylinders, there is provided a fixed rail 988 supported on a fixed stand 989. The cylinder housing includes upper and lower guide plates 990, 992 which slide along the fixed rail 988 to support the cylinder housings in the vertical direction. Rod 966 controls the horizontal movement of the motor. To move the pistons in the respective cylinders, there is provided a double acting valving unit 970 including valves 965-1 to 965-8 schematically shown in FIGURE 39. These valves are controlled by the binary logic on bit No. 1 to bit No. 8 of the binary word
indicating the amount of translation for element 910. Flexible
conduits 972 are the ON or the YES fluid conduits, whereas the
flexible conduits or couplings 974 are the OFF or NO conduits.
A selected binary word indicating the movement of element 910
controls the valves shown in FIGURE 39 to control fluid pressure
to the various cylinders shown in FIGURE 37. By the illustrated
example, the amount of movement of element 910 corresponds to
the magnitude of the binary number in the 8 bit word directed
to the valving unit 970. Element 910 is translated in accordance
with the binary word used as one coordinate (X1) in locating the
cut position of table 750 with respect to cutter 810a. The operation of the three motors 900-904 (X1, X2 and Y) duplicates the
located cut position provided as three binary words (8 bits).
The three motors are moved in accordance with the relationship
of the pins and grooves needed to locate the upper surface of
table 750 for cutting. Primarily, the cut locating mechanism
involves three translating devices which are translated in accordance with the desired ultimate position of leaf L, as indicated
by binary numbers or digital information.

<div align="center">

CONCEPTUAL AND APPARATUS MODIFICATIONS
AND ILLUSTRATIONS

(FIGURES 40, 40A AND 41-43)

</div>

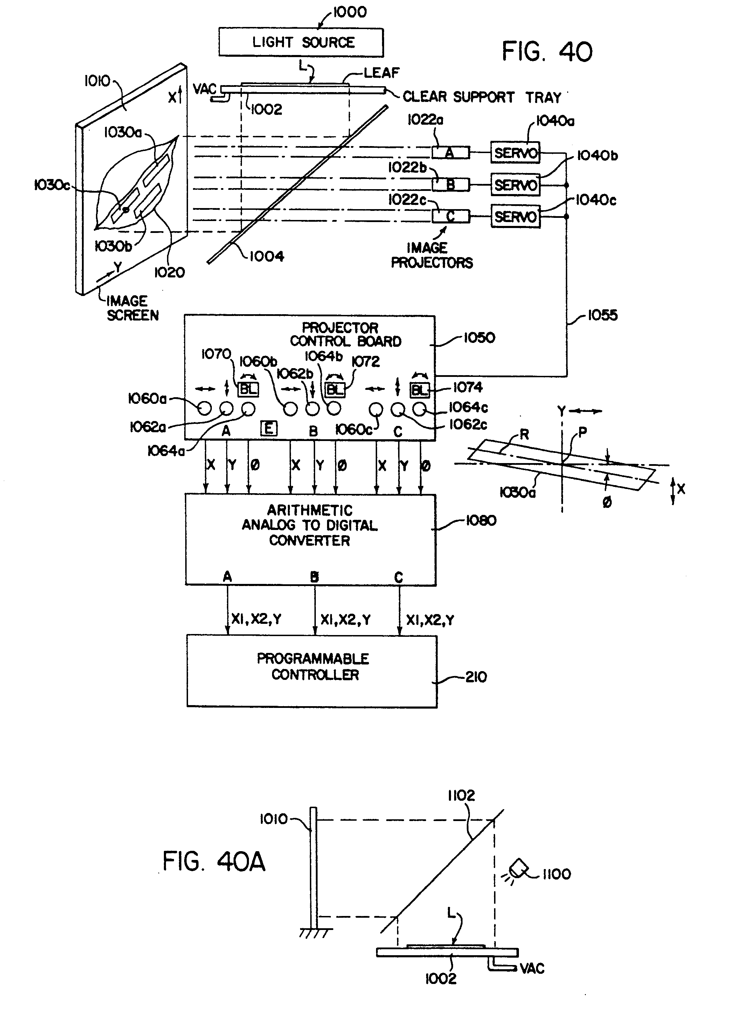

Referring now to FIGURE 40, a conceptual variation of the
system as so far described is illustrated. In this conceptual
concept, an appropriate light 1000 passes light rays through a leaf
support member 1002 onto which a leaf L is supported. The support
member is translucent or transparent so that light rays are transmitted through the support member to an appropriately positioned
one way mirror 1004 which reflects the image of the leaf in oriented fashion onto the display screen 1010. The displayed image 1020
is intersected by light images from projectors 1022a, 1022b,
and 1022c which project images 1030a, 1030b and 1030c corresponding to the cut profile images shown in FIGURE 3 through mirror 1004
onto screen 1010. Servo mechanisms 1040a, 1040b and 1040c are
controlled by a unit 1050 having three sets of manually manipulated
elements 1060a-c, 1062a-c and 1064a-c. For each of the sets of
elements, there is provided a blanking button 1070, 1072, 1074,
respectively. Unit 1050 also has an enter data button E. In
practice, elements 1060a, 1062a, and 1064a are adjusted until the
image or outline 1030a is in a position avoiding all defects in
the image 1020 of leaf L. This movement of elements on the face
of unit 1050 controls servo mechanisms 1040a-c by an appropriate
interconnect indicated as line 1055. After the first outline is
properly positioned at the X, Y and $\emptyset$ coordinates of line R and
point P thereon, the knobs for controlling the next profile 1030b
are manipulated until the image is in the proper position to
create corresponding X, Y and $\emptyset$ coordinates. Thereafter, the
third image 1030c is manipulated to obtain a cut position. If
a third cut can not be made in a non-defect area of the leaf image
1020, the blanking button 1074 is actuated to indicate that no cut
is possible for this third image. The same type of unit 1050 may
be used on the other half of the leaf image to manually manipulate
the cut profiles onto the visually displayed leaf. Thereafter,
the information determined by the position of the control elements
on unit 1050 is transmitted as X, Y and $\emptyset$ to an analog to digital
converter 1080. This digital information is arithmetically converted to coordinate forms acceptable by programmable controller
210 so that the data provided by converter 1080 to programmable
controller 210 is the coordinates X1, X2 and Y as used in the preferred embodiment of the system as so far explained. The system shown in FIGURE 40 illustrates a remotely positioned image determined by the leaf itself into which is fitted the cutting profiles. The information generated is converted to digital information which is indicative of translation that controls the operation of motors 900, 902 and 904 in the illustrated embodiment of the invention. Referring now to FIGURE 40A, the system shown in FIGURE 40 is modified to have a light source 1100 which shines upon leaf L and transmits the front lighted image to the screen 1010 through an optical system 1102. Otherwise, the system of FIGURE 40A operates in accordance with the general system shown in FIGURE 40.

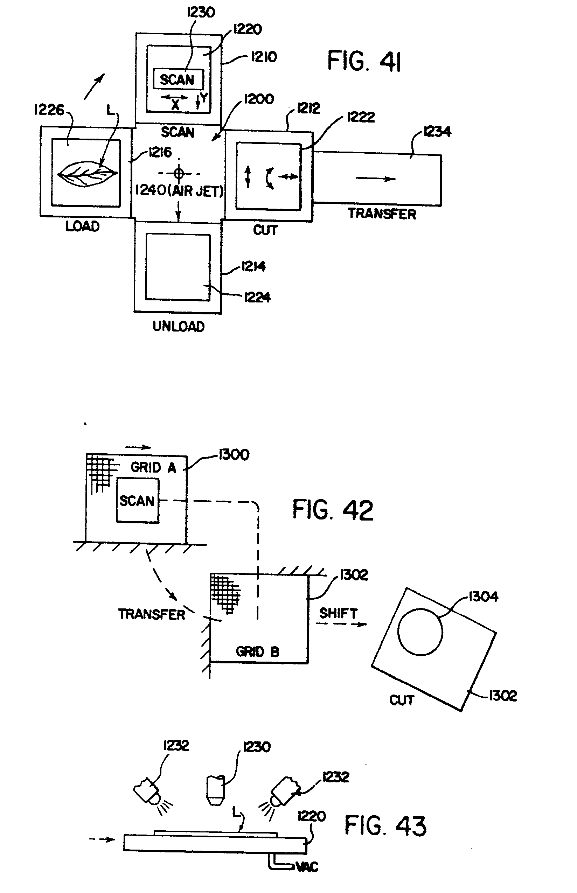

FIGURE 41 schematically illustrates a modified mechanism for processing natural tobacco leaf L. This mechanism includes an indexable turret 1200 movable between scan, cut, unload and load positions and having four supporting platens 1210, 1212, 1214 and 1216. Each of these platens is similar to platen 710 and movably supports cutting boards 1220, 1222, 1224 and 1226 similar to table 750. The cutting tables each include a vacuum surface for holding leaf L onto the cutting table as previously described in the preferred system for processing the natural leaf. A scanning head 1230 is used to scan the leaf L in the X direction as the cutting table is indexed to the Y direction. As shown in FIGURE 43, the leaf L can be illuminated from the front by a plurality of light sources 1232. Of course, light could be positioned under the cutting board 1220 in the scanning position. Turret 1200 is indexed to bring the scanned leaf into the cut position. At that position, the cutting table is indexed and rotated to the various cutting locations and the cutting process is performed as previously described. To transfer the wrapper to a transfer device or vacuum conveyor, platen 1212 as shown in the cutting position, can be indexed to the left to allow transfer of a cut wrapper, as previously described, onto a vacuum belt or conveyor 1234. Thereafter, turret 1200 is indexed to the unload position and air jets 1240 blow the cut leaf from cutting board 1224 as vacuum is released from the cutting table. Following this action, turret 1200 is indexed to the load position where leaf L is spread onto the cutting table 1226. Each of these processes is being performed simultaneously; therefore, during each index, each of the functions previously described is performed at the various illustrated locations. This mechanism illustrates the concept of cutting the leaf on the scanning surface. This does not require reorientation of the leaf onto a new surface as used in the preferred system.

Referring now to FIGURE 42, a conceptual aspect of the present invention is illustrated. In this aspect, a grid A provided on a first member 1300 is fixedly positioned with a grid B on a member 1302. During the scanning operation, the light intensity of the various elements or Pixels on grid A are recorded. Thereafter, the leaf is transferred along the direction indicated by the arrow to grid B. This transfer orients the leaf onto grid B in accordance with the scanning system previously described with respect to grid A. Thus, by utilizing the scanned concept on grid A this concept applies equally to grid B onto which the leaf is oriented. Member 1302 can then be shifted with respect to cutter 1304 to cut a wrapper based upon the position of grid B whereas the scanning has been accomplished with respect to grid A. This is the concept used in the preferred system of the present invention and is shown in FIGURE 42 for illustrative purposes only. Also, this new system can cut different sized wrappers wherein the prior manual system cut only a single sized wrapper from a leaf half.

Claims:

1.　　　　A system for cutting a cigar wrapper from a
natural tobacco leaf having internal color variations
and surface variations and defects detectable by light
rays, said system comprising:　a visible light conducting surface;　means for supporting said leaf in a fixed,
spread condition on said surface;　means for passing
light rays through said surface and said leaf such that
said rays passed have light intensities at identifiable
locations on said surface indicative of the visible
light shielding efficiency of said leaf at said locations
on said surface;　light intensity responsive means for
scanning said locations in sequence;　first reading means
controlled by said scanning means for creating a first
signal when said light intensity of said passed light
rays for one of said identifiable locations is above a
first light intensity;　second reading means controlled
by said scanning means for creating a second signal when
said light intensity of said passed light rays for one
of said identifiable locations is below a second light
intensity;　means for recording at least those identifiable locations wherein there is a transition to or from
said first and second signals;　means for selecting a
wrapper cutting position based upon said recorded locations;　means for transferring said leaf on to a cutting
platen at a known location corresponding to said identifiable locations on said surface;　means controlled by
said selected cutting position for adjusting the relative
position of said cutting platen with respect to a wrapper
cutter spaced from said platen;　means for forcing said
cutter against said platen to cut a wrapper from said
leaf at said adjusted position;　and means for transferring said cut wrapper to a preselected location for
subsequent use.

2.      A system as defined in claim 1, including means
for determining an approximate average of said light
intensities for said identifiable locations and means
for changing said second light intensity in response
to said average.

3.      A system as claimed in claim 1 or 2, wherein
said light conducting surface is formed by a foraminate,
visible light ray conducting, leaf receiving element
having a first leaf supporting surface with an area and
shape capable of receiving said leaf in a spread
condition and a generally coterminous second surface and
including means for creating a positive pressure differ-
ential from said first surface to said second surface
whereby said spread leaf is held against said first
surface.

4.      A system as claimed in claim 3, wherein said
pressure creating means includes a vacuum chamber communicating with said second surface.

5.      A system as claimed in claim 1 or 2, wherein
said light conducting surface is foraminate and including
a vacuum means for holding said leaf against said surface.

6.      A system as claimed in any one of the preceding
claims, including means for moving said light conducting
surface and supported leaf with respect to said light
intensity responsive means.

7.      A system as claimed in any one of the preceding
claims, wherein said cutting platen is a flat member
having a leaf supporting surface, openings in said leaf
supporting surface and a coterminous vacuum chamber
communicated with said leaf supporting surface by said openings and means for selectively applying a vacuum to said coterminous vacuum chamber.

8.        A system as claimed in any one of the preceding claims, wherein said light conducting surface is translucent.

9.        A system for cutting a cigar wrapper from a natural tobacco leaf having internal color variations and surface variations and defects detectable by light rays, said system comprising:  means for supporting said leaf in a fixed spread condition on a surface; means for passing energy rays through said surface and said leaf, said rays having an energy susceptible to variations in transmitted intensity by the shielding effect of said leaf and imperfections or color variations therein;  a sensing means spaced from said leaf for sensing the intensity of energy transmitted through finite, identifiable locations on said surface;  means for constructing a digital profile representative of the energy intensity at said identifiable locations;  means for selecting a cutting position based upon said profile; means for transferring said leaf to a cutting platen; means for adjusting the position of said cutting platen and a wrapper cutter based upon said selected cutting position;  means for forcing said cutter against said platen to cut said wrapper;  and, means for transferring said cut wrapper to a preselected location for subsequent use.

10.       A system for cutting a cigar wrapper of a given contour from a natural tobacco leaf having two large area surfaces and having internal color variations and surface variations and defects detectable by light rays, said system comprising:  a light conducting surface;

means for supporting said leaf in a fixed, spread condition on said surface; means for passing light rays through said light conducting surface and said leaf to create an image of said leaf in variations of light intensities based upon the shielding effect of said leaf and surface variations on said two surfaces thereof; means selecting a cutting position on said leaf by comparing said wrapper contour with said image; means for recording said selected cutting position; means for locating said leaf on a cutting platen; means responsive to said recorded position for positioning a cutter at said recorded position on said leaf on said platen; means for cutting said wrapper by said cutter at said recorded position; and, means for then transferring said cut wrapper to a selected location for subsequent use.

11. A system as claimed in claim 10, wherein said light conducting surface is foraminate and including a vacuum means for holding said leaf against said surface.

12. A system as claimed in claim 10 or 11, wherein said cutting platen is a flat member having a leaf supporting surface, openings in said leaf supporting surface and a coterminous vacuum chamber communicated with said leaf supporting surface by said openings and means for selectively applying a vacuum to said coterminous vacuum chamber.

13. A system as claimed in claim 10, 11 or 12, wherein said light conducting surface is translucent.

14. A method of cutting a cigar wrapper from a natural tobacco leaf, said method comprising the steps of:

(a)   supporting said leaf in a spread condition;

(b)   passing light rays through said leaf to
create light intensities at identifiable locations
indicative of the visible light shielding efficiency
of said leaf at said locations;

(c)   reading the light intensity at said identifiable
locations;

(d)   recording at least said locations wherein said
light intensity shifts to a given high or low value;

(e)   selecting a wrapper cutting position based
upon said recorded locations;  and,

(f)   cutting a wrapper from said selected position
of said leaf.


15.      A method of cutting a cigar wrapper with a given
contour from a natural tobacco leaf having two large area
surfaces, said method comprising the steps of:

(a)   spreading said leaf;

(b)   supporting said leaf in a spread condition;

(c)   passing light rays through said surfaces of
said leaf to create an image of said leaf in variations
of light intensities based upon the shielding effect of
said leaf and surface variations on said two surfaces
thereof;

(d)   selecting a cutting position on said leaf by
comparing said wrapper contour with said image;

(e)   recording said selected cutting position;

(f)   positioning a cutter at said recorded position
on said leaf;  and,

(g)   cutting said wrapper by said cutter at said
recorded position.


16.      An apparatus for detecting the general outline
and abrupt surface variations of a flaccid sheet-like
workpiece formed from at least a portion of a natural leaf, said apparatus comprising: a foraminate, visible light ray conducting workpiece receiving element having a first workpiece supporting surface with an area and shape capable of receiving said workpiece in a spread condition and a coterminous second surface; means for creating a positive pressure differential from said first surface to said second surface whereby a workpiece is held against said first surface; a light source means on one side of said element for passing light rays through said workpiece receiving element and through said workpiece, with the transmitted light intensity at the other side of said element being indicative of the outline and surface conditions of said workpiece; a light sensitive transducer means for receiving said light rays on said other side of said element, said transducer means including a plurality of light responsive transducers juxtapositioned along a given path and means for directing light rays from only a finite linear portion of said workpiece receiving element to said given path; means for incrementally changing said finite linear portion sequentially from position-to-position along said workpiece; means for reading the conditions of said transducers at each position of said finite linear portion; means for sensing when one of said conditions of said transducers exceeds a first light intensity condition; and, means for sensing when one of said conditions of said transducers is less than a second light intensity condition.

17. A method of cutting a cigar wrapper from a natural tobacco leaf having a major width, a length and two generally parallel surfaces by a cigar wrapper cutter, said method comprising the steps of:

(a) supporting said leaf in a spread condition;

(b) passing light rays through said leaf;

(c)   sensing the shielded light intensity at a
selected longitudinal position and at transversely
spaced incremented locations extending a transverse
distance greater than the major width of said leaf;

(d)   repeating step (c) at a next adjacent longitudinal position on said leaf until said locations
have been sensed over the length of said leaf;

(e)   converting said light intensity of each of
said locations into a digital signal representative
of the general light intensity at each of said locations;

(f)   sequentially storing at least some of said
digital representative signals;

(g)   selecting a cutting position based upon said
stored signals;

(h)   creating cut coordinates corresponding to said
selected cut position;

(i)   transferring said leaf to a cutting platen;

(j)   converting said cut coordinates into relative
movement of said platen and cutter to locate said cutter
at said selected cut position on said leaf;   and,

(k)   cutting said wrapper from said leaf at said
selected position.

18.      An apparatus for detecting the general outline
and abrupt surface variations of a flaccid sheet-like
workpiece formed from at least a portion of a natural
tobacco leaf, said apparatus comprising:   a foraminate,
light ray conducting workpiece receiving element having
a first workpiece supporting surface with an area and
shape capable of receiving said workpiece in a spread
condition and a coterminous second surface;   means for
creating a positive pressure differential from said
first surface to said second surface whereby a workpiece
is held against said first surface;   a light source
means on one side of said element for passing light rays through said workpiece receiving element and through said workpiece, with the transmitted light intensity at the other side of said element being indicative of the shielding effect of said receiving element and said workpiece; a series of light intensity measuring units extending in a first direction across said receiving element and spaced from said first surface; means for creating a digital signal indicative of the shielded light intensity at each of said units; means for recording said digital signals; means for shifting the relative position of said units and said leaf in a direction generally orthogonal to said first direction; means for recording said digital signals at said shifted relative position; and means for creating a representation of said general outline and abrupt surface variations based upon said recorded signals.

19.    . An apparatus as claimed in claim 18, wherein said shifting means includes means for shifting said receiving element with respect to said units.

20.    An apparatus as claimed in claim 19, wherein said light source is an elongated light and including means for fixedly mounting said light adjacent said second surface and aligned with said units.

21.    An apparatus as claimed in claim 18 or 19, wherein said light source is an elongated light and including means for mounting said light adjacent said second surface and fixedly with and generally parallel to said units.

22.    An apparatus as claimed in claim 18, 19, 20 or 21, wherein said means for creating said digital representative signals includes means for creating said

signals in series.

23.      An apparatus as claimed in claim 22, including
sensing means for indicating when said series of signals
has been created and means responsive to said sensing
means for activating said shifting means.

24.      An apparatus for detecting the general outline
and abrupt surface variations of a flaccid sheet-like
workpiece formed from at least a portion of a natural
tobacco leaf, said apparatus comprising:  a foraminate,
light ray conducting workpiece receiving element having
a first workpiece supporting surface with an area and
shape capable of receiving said workpiece in a spread
condition and a coterminous second surface;  means for
creating a positive pressure differential from said first
surface to said second surface whereby a workpiece is
held against said first surface;  a light source means
on one side of said element for passing light rays
through said workpiece receiving element and through
said workpiece, such that the transmitted light intensity
at the other side of said element is indicative of the
shielding effect of said receiving element and said workpiece;  a series of light intensity measuring units
extending in a first direction across said receiving
element and spaced from said first surface;  scanning
means for creating a scanned series of digital signals
indicative of the shielded light intensity at each of
said units;  means for cycling said scanning means through
a cycle;  means for shifting the relative position of said
units in a direction generally orthogonal to said first
direction;  scan starting means responsive to said shift-
ing means for activating said cycling means through a
successive scanning cycle;  means for recording said
digital signals at said relative positions during each of said scan cycles;  and means for creating a representation of said general outline and abrupt surface variations based upon said recorded signals.

25.      An apparatus as claimed in claim 24, including means responsive to the end of each scanning cycle for allowing operation of said scan starting means.

26.      An apparatus as claimed in claim 24 or 25, including a digital counter means for counting each of said digital signals, a decoder means for creating a decoded digital signal indicative of each of said signals and means for using said decoded signal and said digital signal for creating said representation.

27.      An apparatus as claimed in claim 26, including a second digital counter means for counting each of said shifts in said relative position and a second decoder means for creating a second digital signal indicative of each of said shifts and means for using said second decoded signal and said digital signals after each said shift for creating said representation.

28.      An apparatus as claimed in any one of claims 24 to 27, wherein said digital signal scanning means includes means for creating a first analyzed digital signal when the light intensity of a measuring unit reads a value exceeding a preselected level, a second analyzed digital signal when the light intensity of a measuring unit reads a value less than a preselected value and means for creating a third analyzed digital signal when the light intensity of a measuring unit is between said preselected values.

29.      A method of making a flat profile having a preselected shape from a natural leaf having surface variations and defects detectable by light rays, said method comprising the steps of:

(a) supporting said leaf in a spread condition on a support surface at a random location;

(b) creating a leaf profile including an edge outline and surface imperfections;

(c) locating at least one cut location within said outline and excluding said imperfections; and,

(d) cutting said profile from said leaf at a cut position corresponding to said cut location.

30. A method as claimed in claim 29, including the additional steps of:

(e) recording said cut location as it relates to said support surface;

(f) transferring said leaf to a cutting surface at a location corresponding to said random location; and,

(g) cutting said profile at a cut position on said cutting surface corresponding to said cut location recorded with respect to said support surface.

31 A method of making a flat profile having a preselected shape from a natural leaf having surface variations and defects detectable by light rays, said method comprising the steps of:

(a) supporting said leaf in a spread condition on a first surface and at a random position;

(b) creating a leaf profile including an edge outline and surface imperfections;

(c) locating at least one cut location within said outline and excluding said imperfections;

(d) recording said cut location with respect to its position on said first surface;

(e) locating a cutting member in a fixed position with respect to said first surface;

    (f)   transferring said leaf to said cutting member in a location corresponding to said random location;

    (g)   adjusting the position of said cutting member with respect to a cutter until said cutter is positioned with respect to said cutting member at a position corresponding to said recorded location;  and,

    (h)   cutting said profile from said leaf by said cutter at said adjusted position of said cutting member.


32.      A system of making a flat profile having a preselected shape from a natural leaf having surface variations and defects detectable by light rays, said system comprising:

    (a)   means for supporting said leaf in a spread condition on a support surface at a random location;

    (b)   means for creating a leaf profile including an edge outline and surface imperfections;

    (c)   means for locating at least one cut location within said outline and excluding said imperfections; and,

    (e)   means for cutting said profile from said leaf at a cut position corresponding to said cut location.


33.      A system as claimed in claim 32, further including:

    (f)   means for recording said cut location as it relates to said support surface;  and,

    (g)   means for transferring said leaf to a cutting surface at a location corresponding to said random location where said profile is cut.


34.      A system of making a flat profile having a preselected shape from a natural leaf having surface variations and defects detectable by light rays, said system comprising:

(a)  means for supporting said leaf in a spread condition on a first surface and at a random position;

(b)  means for creating a leaf profile including an edge outline and surface imperfections;

(c)  means for locating at least one cut location within said outline and excluding said imperfections;

(d)  means for recording said cut location with respect to its position on said first surface;

(e)  means for locating a cutting member in a fixed position with respect to said first surface;

(f)  means for transferring said leaf to said cutting member in a location corresponding to said random location;

(g)  means for adjusting the position of said cutting member with respect to a cutter until said cutter is positioned with respect to said cutting member at a position corresponding to said recorded location;  and,

(h)  means for cutting a wrapper from said leaf by said cutter at said adjusted position of said cutting member.

35.     An apparatus for cutting a profile having a preselected shape from a sheet material at a given location, said apparatus comprising a cutting platen having an upper generally flat cutting surface and a lower generally flat support surface, said surfaces being generally parallel;  means on said cutting surface for supporting said material;  a cutter spaced from said cutting surface and having said preselected shape;  means for moving said platen in a direction parallel to said surfaces until said cutter is aligned at said given location on said material;  and means for forcing said cutter against said surface along an axis generally orthogonal to said surfaces, character- ised by:  a fixed anvil means having a flat force reaction surface parallel to and facing said flat support surface;  means for locating said anvil surface in alignment with said axis;  and means for allowing said support surface to bear against said reaction surface at least after said cutter engages said cutting surface.

36.     Apparatus as claimed in claim 35, wherein said reaction surface engages said support surface as said platen is moving.

37.     Apparatus as claimed in claim 35 or 36, wherein said reaction surface forms the moving support means for said platen.

38.     A system for cutting a cigar wrapper from a natural tobacco leaf, said system comprising:  means defining a visible light conducting surface;  means for supporting said leaf in a fixed, spread condition on said surface;  means passing light rays through said surface and said leaf, said rays passed having light intensities at identifiable locations on said surface indicative of the visible light shielding efficiency of said leaf at
said locations on said surface;   light intensity responsive means for scanning said locations in sequence;
first reading means controlled by said scanning means
for creating a first signal when said light intensity
of said passed light rays for one of said identifiable
locations is above a first light intensity;   second
reading means controlled by said scanning means for
creating a second signal when said light intensity of
said passed light rays for one of said identifiable
locations is below a second light intensity;   means for
recording at least those identifiable locations wherein
there is a transition to or from said first and second
signals;   means for selecting a wrapper cutting position
based upon said recorded locations;   means for transferring said leaf on to a cutting platen at a known location
corresponding to said identifiable locations on said
surface;   means controlled by said selected cutting position for adjusting the relative position of said cutting
platen with respect to a wrapper cutter spaced from said
platen;   means for forcing said cutter against said platen
to cut a wrapper from said leaf at said adjusted position;
and means for transferring said cut wrapper to a preselected location for subsequent use, characterised by:   said
platen having a generally flat cutting surface and means
for supporting said leaf and including a generally fixed
anvil means for engaging said platen from a first
direction generally perpendicular to said cutting surface
and spaced from said cutting surface and said forcing
means including means for forcing said cutter against
said cutting surface and against the reactive force of
said anvil.

39.     A system for cutting a cigar wrapper of a given
contour from a natural tobacco leaf having two large area surfaces, said system comprising: means defining a light conducting surface; means for supporting said leaf in a fixed, spread condition on said surface; means for passing light rays through said light conducting surface and said leaf to create an image of said leaf in variations of light intensities based upon the shielding effect of said leaf and surface variations on said two surfaces thereof; means selecting a cutting position on said leaf by comparing said wrapper contour with said image; means for recording said selected cutting position; means for locating said leaf on a cutting platen; means responsive to said recorded position for positioning a cutter at said recorded position on said leaf on said platen; means for cutting said wrapper by said cutter at said recorded position; and, means for then transferring said cut wrapper to a selected location for subsequent use, characterised by: said platen having a generally flat cutting surface and means for supporting said leaf and including a generally fixed anvil means for engaging said platen from a first direction generally perpendicular to said cutting surface and spaced from said cutting surface and said forcing means including means for forcing said cutter against said cutting surface and against the reactive force of said anvil.

40. A system as claimed in claim 38 or 39, wherein said anvil includes a reaction surface parallel to said cutting surface and said platen includes a lower surface parallel to said reaction surface, and means for allowing said lower surface to slide along said reaction surface during movement of said platen.

41.　　　A device for converting a succession of analog
voltage signals representative of the light intensity
of light from and controlled by a corresponding succession
of finite surface locations of a natural leaf into a
white, black or gray digital signal for each given
location, said device comprising:　means for creating a
white digital signal when an analog voltage signal at a
given location exceeds a threshold voltage corresponding
to a given light intensity value;　averaging means for
creating a substantially continuously updated datum
voltage by averaging at least a majority of previous
analog signals of said succession of locations;　means
for reducing said datum voltage to an offset datum
voltage;　means for comparing said analog signal for a
given location with said offset datum voltage;　means for
creating a black digital signal when said analog signal
for said given location is less than said offset datum
voltage;　and, means for creating a gray digital signal
in the absence of said white and black signals for a
given location.

42.　　　A device as claimed in claim 41, including means
for amplifying said analog voltage signal and said averaging means and black signal creating means being operative
on said amplified analog signal.

43.　　　A device as claimed in claim 41 or 42, including
means for excluding said analog signal for a given location from said averaging means when said analog signal
creates said white digital signal.

44.　　　A device as claimed in claim 41, 42 or 43,
including means for excluding said analog signal for a
given location from said averaging means when said analog
signal creates said black digital signal.

45.     A method for converting a succession of analog
voltage signals representative of the light intensity
of light from and controlled by a corresponding succession
of finite surface locations of a natural leaf into a
white, black or gray signal for each given location,
said method comprising the steps of:

(a)  creating a white digital signal when an analog
voltage signal at a given location exceeds a threshold
voltage corresponding to a given light intensity value;

(b)  creating a substantially continuously updated
datum voltage by averaging at least a majority of previous analog signals of said succession of locations;

(c)  reducing said datum voltage to an offset datum
voltage;

(d)  comparing at least the analog signal for a given
location which does not exceed said threshold voltage
with said offset datum voltage;

(e)  creating a black digital signal when said analog
signal for a given location is less than said offset
datum voltage;  and,

(f)  creating a gray digital signal in the absence
of said white and black signals for a given location.


46.     A method as claimed in claim 45, including the
additional step of:

(g)  excluding said analog signal for a given location
from said datum voltage creating step when said analog
signal exceeds said threshold voltage.


47.     A method as claimed in claim 46, including the
additional step of:

(h)  excluding said analog signal for a given location
from said datum voltage creating step when said analog
signal creates a black digital signal.

48.     An apparatus for cutting cigar wrappers with a
selected profile from a natural tobacco leaf, said
apparatus comprising:  vacuum means for supporting said
leaf in a spread condition and in a random position on a
leaf receiving member;  means for selecting at least one
cut position on said leaf and oriented with respect to
said leaf receiving member;  a cutting platen including
a leaf support surface and means for creating a vacuum
at said leaf support surface;  means for positioning
said platen in a known leaf receiving position with
respect to said leaf receiving member;  transfer means
for transferring said leaf from said leaf receiving
member to said leaf support surface when said cutting
platen is in said leaf receiving position;  a wrapper
cutter having said selected profile;  means for shift-
ing said platen from said leaf receiving position to a
leaf cutting position with said support surface of said
platen spaced from said wrapper cutter;  means for orienting said platen relative to said cutter to a position
with said cutter aligned with said selected cut position;
means for forcing said cutter against said platen whereby
a wrapper is cut and separated from said leaf;  and means
for then transferring said cut and separated wrapper to
a selected location for subsequent use.

49.     An apparatus as claimed in claim 48, wherein said
cutter forcing means is a piston in a cylinder and means
for directing fluid to said cylinder.

50.     An apparatus as claimed in claim 49, including
means for shifting said cutter with respect to said
piston between a retracted non-cutting position and an
extended wrapper cutting position.

51.     An apparatus as claimed in claim 50, wherein

said shifting means is a cam coacting between said
cutter and said piston and means for shifting said
cam between a first position with said cutter adjacent
said piston and a second position with said cutter
extended from said piston.

52.     An apparatus as claimed in any one of claims
48 to 51, including an anvil generally aligned with
said cutter and means on said platen for engaging said
anvil as said cutter is forced against said platen.

53.     An apparatus for cutting a selected one of a
plurality of profiles from a sheet material comprising:
means for supporting said sheet material in a spread
condition on a cutting surface;  a cutting member having a plurality of cutters each with a cutting profile
corresponding to one of said plurality of profiles;
means for forcing said cutting member toward said cutting surface and means for shifting each of said cutters
with respect to said cutting member between a retracted
non-cutting position and an extended cutting position.

54.     An apparatus as claimed in claim 53, wherein
each of said shifting means is a cam coacting with one
of said cutters and said cutter member and means for
shifting said cam between a first position with said
cutter adjacent said member and a second position with
said cutter extended from said member.

55.     An apparatus as claimed in claim 53 or 54,
wherein said member is a piston and said forcing means
includes fluid means for forcing said piston toward said
cutting surface.

56.      A device for translating and rotating a cutting
platen for a flat workpiece with respect to a recipro-
cable cutter, said device comprising first, second and
third guide ways on said platen, said first and second
guide ways extending generally in a first direction
and said third guide way extending in a second direction
at a known angle to said first direction;  first, second
and third drive elements slidably received in said first,
second and third guide ways respectively;  first drive
means for shifting said first drive element in a drive
direction generally transverse to said first guide way;
second drive means for shifting said second drive element
in a drive direction generally transverse to said second
guide way;  third drive means for shifting said third
drive element in a drive direction generally transverse
to said third guide way;  and control means for shift-
ing said first, second and third drive means a selected
distance in said drive directions to cause corresponding
movement of said platen with respect to said cutter.

57.      A device as claimed in claim 56, wherein said
known angle is approximately 90°.

58.      An apparatus as claimed in claim 56 or 57,
wherein at least said first and second drive means
includes a drive member movable by a binary fluid motor
including a series of axially aligned fluid actuated
units, each of said units including a cylinder and an
extendable piston having a controlled stroke with respect
to said cylinder, said respective strokes having a known
binary distance relationship, and means for interconnecting said fluid units of each fluid motor to cause movement
of said driven members rectilinear distances corresponding
to the summation of the strokes of the extended pistons
of said units of each fluid motor.

- 22 -

0004170

59.     An apparatus as claimed in claim 56, 57 or 58,
including means separate from said drive elements for
vertically supporting said platen during movement of
said platen.

60.     An apparatus as claimed in claim 59, wherein
said drive elements are upwardly extending pins engag-
ing said guide ways in only a transverse direction.

61.     A device for cutting a piece from a flat workpiece at a location identified by two or more binary
coded numbers, said device comprising:  a cutter;  a
cutting platen having an upper workpiece supporting
cutting surface and a lower portion;  a plurality of upstanding shiftable members;  guide means on said platen
adjacent said lower portion for loosely receiving said
members;  means for moving said members in accordance
with said coded numbers;  and means for forcing said
cutter against said cutting surface.

62.     A device as claimed in claim 61, wherein said
guide means includes first, second and third guide ways
on said platen, said first and second guide ways extend-
ing generally in a first direction and said third guide
way extending in a second direction at a known angle to
said first direction wherein said shiftable members are
first, second and third drive elements slidably received
in said first, second and third guide ways respectively
and wherein said moving means includes first drive means
for shifting said first drive element in a drive direct-
ion generally transverse to said first guide way, second
drive means for shifting said second drive element in a
drive direction generally transverse to said second guide
way, and third drive means for shifting said third drive
element in a drive direction generally transverse to said
third guide way.

63.     A device as claimed in claim 62, wherein at least said first and second drive means includes a drive member movable by a binary fluid motor including a series of axially aligned fluid actuated units, each of said units including a cylinder and an extendable piston having a controlled stroke with respect to said cylinder, said respective strokes having a known binary distance relationship, means for interconnecting said fluid units of each fluid motor to cause movement of said driven member a rectilinear distance corresponding to the summation of the strokes of the extended pistons of said units of each fluid motor and means for extending said pistons of said units for each fluid motor in response to said coded numbers.

64.     A device as claimed in claim 62 or 63, wherein said third drive means includes a drive member movable by a third fluid motor including a series of axially aligned fluid actuated units, each of said units of said third fluid motor including a cylinder and an extendable piston having a controlled stroke with respect to said cylinder, said strokes of said units of said third fluid motor having a known binary distance relationship, means for interconnecting said fluid units of said third fluid motor to cause movement of said third driven member a rectilinear distance corresponding to the summation of the strokes of the extended pistons of said units of said third fluid motor and means for extending said pistons of said units for said third fluid motor in response to a selected coded number.

65.     A device as claimed in claim 64, including means for locating said third fluid motor in a selected intermediate position between cuts by said cutter.

66.      A device as claimed in claim 62, 63 or 64,
including means for locating at least one of said drive
means in an intermediate position between cuts by said
cutter.

67.      A device as claimed in any one of claims 61 to
64, including means for locating at least one of said
shiftable members in an intermediate position between
cuts.

68.      A device as claimed in any one of claims 61 to
67, including vacuum means for supporting said workpiece
on said cutting surface.

69.      A device as claimed in claim 68, wherein said
cutting platen includes a plenum chamber below said
cutting surface, means for directing a vacuum to said
plenum chamber, said chamber comprising a plurality of
spaced subchambers and apertures extending between said
cutting surface and one of said subchambers.

70.      A device for moving a cutting platen with respect
to a vertically movable cutter to a cutting position,
said platen having an upper generally flat cutting surface
for supporting a generally flat sheet and a lower portion,
said device comprising:  two upstanding shiftable members;
first and second generally parallel guide ways on said
platen and adjacent to said lower portion, said guide
ways being generally parallel and dimensioned to allow
translation of one of said members in each of said guide
ways;  means for extending said members into said guide
ways;  and, first and second drive means for shifting
said members preselected, independent distances along
generally parallel paths to impart both rotation and
translation to said platen.

71.     A device as claimed in claim 70, wherein said first and second drive means each includes a fluid motor, a drive member movable by said fluid motor and means for moving each of said fluid motors a distance determined by a multi-bit digital signal.

72.     A device as claimed in claim 71, wherein said fluid motors each include a series of axially aligned fluid actuation units, each of said units including a cylinder and an extendable piston having a controlled stroke with respect to said cylinder, said respective strokes having a known binary distance relationship, means for interconnecting said fluid units of each fluid motor to cause movement of said shiftable members to a rectilinear position corresponding to the summation of the strokes of the extended pistons of said units of each of said fluid motors and means for extending selected ones of said pistons in response to the bits of said multi-bit digital signal.

73.     An apparatus for cutting cigar wrappers from a natural tobacco leaf, said apparatus comprising:  vacuum means for supporting said leaf in a spread condition and in a random position on a leaf receiving member;  means for selecting at least one cut position on said leaf and oriented with respect to said leaf receiving member; a cutting platen including a leaf support surface and means for creating a vacuum at said leaf support surface; means for positioning said platen in a known leaf receiving position with respect to said leaf receiving surface;  transfer means for transferring said leaf from said leaf receiving surface to said leaf support surface when said cutting platen is in said leaf receiving position;  means for shifting said platen from a leaf receiving position to a leaf cutting position with said surface of said platen spaced from a wrapper cutter; means for orienting said platen with respect to said cutter to a position with said cutter aligned with said selected cut position; means for forcing said cutter against said platen whereby a wrapper is cut and separated from said leaf; and means for then transferring said cut and separated wrapper to a selected location, characterised in that said platen orienting means includes a guide mechanism for said platen, said guide mechanism comprising first, second and third guide ways on said platen, said first and second guide ways extending generally in a first direction and said third guide way extending in a second direction at a known angle to said first direction; first, second and third drive elements slidably received in said first, second and third guide ways respectively; first drive means for shifting said first drive element in a drive direction generally transverse to said first guide way; second drive means for shifting said second drive element in a drive direction generally transverse to said second guide way; third drive means for shifting said third drive element in a drive direction generally transverse to said third guide way; and control means for shifting said first, second and third drive means a selected distance in said drive directions to cause corresponding movement of said platen with respect to said cutter.

74. An apparatus as claimed in claim 73, wherein at least said first and second drive means includes a drive member movable by a binary fluid motor including a series of axially aligned fluid actuated units, each of said units including a cylinder and an extendable piston having a controlled stroke with respect to said cylinder, said respective strokes having a known binary distance relationship, and means for interconnecting said fluid units of each fluid motor to cause movement of said driven member a rectilinear distance corresponding to the summation of the strokes of the extended pistons of said units of each fluid motor.

75.     A digital device for storing into a memory unit a positionally oriented digital representation of the outline and distinct light detectable surface variations of a natural tobacco leaf having two generally parallel large area surfaces with light detectable surface variations, said device comprising: means for supporting a said leaf in a spread condition on a surface; means for illuminating a said leaf supported on said surface to create an emitted light intensity pattern of selected locations on said surface indicative of the non-existence of a leaf, the existence of a leaf and light intensity of the surface of said leaf; measuring the emitted light intensity at said selected locations; means for creating a first digital signal when said light intensity at a location is above a selected first level; means for creating a second digital signal when light intensity at a location is below a selected second level; means for creating a third digital signal in the absence of said first and second signals; scanning means for reading a succession of said measured light intensities for locations in a given path across said surface in a first direction; means for shifting said path in a second direction generally orthogonal to said first direction; means for creating a digital address for each of said locations; means for creating a transition digital signal when a first, second or third digital signal for one location changes to another signal in a next successive location; means for transmitting said digital transition signal identifying said other signal and a digital address of said one location to said memory unit.

76.     A digital device as claimed in claim 75, including in combination said memory unit; means for accumulating the number of transition signals during said successive paths;  and, means for storing in a first
area of said memory unit the total number of transition
signals accumulated in each successive path in said
memory unit, said total number including accumulated
transition signals in prior paths.

77.      A digital device as claimed in claim 76, including means for storing in said second area of said memory
unit, in succession, each of said digital transition
signals and said digital address for each location of
said transition signals.

78.      A device for creating a series of digital signals
indicative of the profile and certain surface variations
of a natural leaf, said device comprising:  means for
supporting said leaf in a spread condition on a member;
means for sensing light emitted from a succession of
locations on said member;  means for scanning said emitted
light at said locations in succession in a given path
and then in successive paths parallel to said given path
until the light emitted from all locations on said member
and covered by a said leaf thereon in use are scanned;
first means for creating a first digital pattern signal
when the light intensity of the emitted light from a
scanned location is above a preselected level;  second
means for creating a second digital pattern signal when
the light intensity of the emitted light from a scanned
location is above a selected level less than said preselected level;  means for creating a third digital
pattern signal in the absence of said first and second
digital pattern signals for a scanned location;  means
for creating a transition signal for a location when a
prior digital pattern signal is different from a scanned
digital pattern signal;  means determining the pattern
signal to which said transition signal is made and means for recording said transition and determined pattern
signal in a pattern corresponding to said scanning
pattern.

79.        A method of creating in a digital memory a
positionally oriented digital representation of the outline and distinct light intensity variations of a natural
tobacco leaf having two generally parallel large area
surfaces, said method comprising the steps of:
    (a)    supporting said leaf in a spread condition on
a member;
    (b)    illuminating said leaf to create an emitted
light ray pattern corresponding to the shape and surface
coloration of said leaf;
    (c)    measuring the emitted light intensity at known
locations on said supporting member, said locations
combining to cover said leaf and part of said member;
    (d)    creating a digital representation for each of
said locations, said representation being:
            (i)    a first digital signal when the light
                   intensity for said location is above a
                   selected first level;
           (ii)    a second digital signal when the light
                   intensity for said location is below
                   a selected second level;  or,
          (iii)    a third digital signal in the absence
                   of said first and second digital signals
                   for said location;
    (e)    creating a digital transition signal for each
of said locations wherein said digital representation
shifts from one of said digital signals to another of
said digital signals;  and,
    (f)    storing in said memory each of said digital
transition signals and information identifying the
location thereof.

80.     A method of creating in a digital memory a positionally oriented digital representation of the outline and distinct light intensity variations of a natural tobacco leaf having two generally parallel large area surfaces, said method comprising the steps of:

(a)     supporting said leaf in a spread condition on an oriented light ray transmitting member;

(b)     passing light rays through said leaf and said member;

(c)     measuring the transmitted light intensity at selected locations on said member, said locations combining to generally cover said leaf;

(d)     creating a digital representation for each of said locations, said representation being:

   (i)     a first digital signal when the light intensity for said location is above a selected first level;

   (ii)    a second digital signal when the light intensity for said location is below a selected second level;

   (iii)   a third digital signal in the absence of said first and second digital signals for said location;

(e)     creating a digital transition signal for each of said locations wherein said digital representation shifts from one of said digital signals to another of said digital signals;  and,

(f)     storing in said memory each of said digital transition signals and information identifying the location thereof.

81.     A method as claimed in claim 79 or 80, including creating a digital address code for each of said locations wherein said digital representation shifts from one of said digital signals to another of said digital signals and storing in said memory said digital address code
identifying the location of each of said transition
signals.

82.    A method of selecting the cutting position of a cigar wrapper cutter having a given shape with respect to a natural tobacco leaf using a remotely created leaf image of a given tobacco leaf, which image includes the outline of the given leaf, the position of the leaf stem and the areas with holes in said leaf, said method comprising the steps of:

(a)  providing an outline image of the given cutter shape;

(b)  shifting the outline image with respect to said leaf image until said outline image is in a selected position avoiding the outline, the stem and any hole areas of said leaf image;  and,

(c)  then, creating cutting coordinates indicative of said selected position for use in controlling a cutting position of said cigar wrapper cutter on said given leaf.

83.    A method as claimed in claim 82, including the additional steps of:

(d)  shifting said outline image until said outline image is in a second selected position avoiding said first mentioned selected position, the outline, the stem and any hole areas of said leaf image;  and,

(e)  then, creating second cutting coordinates indicative of said second selected position for use in controlling a second cutting position of said cigar wrapper cutter on said given leaf.

84.    A method of selecting the cutting position of a cigar wrapper cutter with respect to a natural tobacco leaf using data stored in a digital memory and including information indicative of the outline of said leaf, the position of the leaf stem, the outline of areas with holes in said leaf and the position of dark areas on said leaf, said method comprising the steps of:

(a)   providing a digital representation of the general cutter shape;

(b)   creating a safe line adjacent to and spaced from said leaf stem;

(c)   placing said cutter shape representation in a first position adjacent to said safe line;

(d)   checking to determine if said representation intersects said leaf outline or includes part or all of one of said outlined hole areas;

(e)   if no intersection or hole area inclusion is found, developing the coordinates of said first position;

(f)   if there is an intersection or hole area inclusion for the first position, shifting said shape representation a selected distance to a next position longitudinally along said safe line and repeating steps (d) and (e);

> (i)   repeating step (f) until there is no intersection or hole area inclusion;

> (ii)   developing the coordinates of the first next position free of said intersection and hole areas;   and

(g)   using said created coordinates for controlling the cutter position on said leaf of said cigar wrapper cutter.

85.      A method as claimed in claim 84, including after developing the coordinates of said first position or said first next position free of said intersection or hole areas the following steps:

(a)   enlarging said leaf outline to include said cutter shape representation at said developed coordinates to give a modified leaf outline;

(b)   shifting said cutter shape representation to another position within said modified leaf outline if possible;

(c) checking to determine if said cutter shape representation includes part or all of one of said outlined hole areas;

(d) if at least part or all of one of said outlined hole areas are within said another position, shift said shape representation and repeat step (c);

(e) repeating steps (c) and (d) until there is no inclusion of part or all of one of said hole outlined areas and then creating coordinates of said another position; and,

(f) using said created coordinates at said another position for controlling another cutting position on said leaf of said cigar wrapper cutter.

86. A method as claimed in claim 84 or 85, wherein step (a) comprises providing a vectorized digital representation of the general cutter shape.

FIG. 1

FIG. 2

FIG. 3

SPREAD LEAF ON VACUUM SUPPORT SURFACE — 40

LINEAR INDEXING INCREMENTED SCAN OF LIGHT PASSING THROUGH LEAF AT INDENTIFIABLE LOCATIONS — 42

INTENSITY AT LOCATION — 44

CONVERT LOCATION ANALOG DATA TO DIGITAL DATA — 50

SEQUENCER — 60

DIGITAL DATA — 72

SEQUENCE STORAGE UNIT FOR DIGITAL SCAN DATA — 70

LOCATION DATA — 74

LOCATION DATA — 46

INCREMENT — 469

TRANSFER LEAF TO CUT TABLE — 90

LOCATE CUTS X Y Ø — 80

LOCATE CUTTER WITH TABLE FOR CUT #1 — 102 | DIGITAL TO ANALOG MOVEMENT — 100

GENERATE COORDINATES OF CUTS XI, X2, Y — 82

92

FIG. 4

CUT WRAPPER — 104

REMOVE CUT WRAPPER FROM TABLE — 106

STORE CUT WRAPPER — 108

CUT #2···N — 110

PROGRAM CONTROLLER — 210

SCAN MECH. — 200

92

HEWLETT PACKARD HP-2112

DIGITAL COMPUTER — 240

DIRECT ACC. MEM. — 220

LEAF TRANSFER MECH. — 202

LOCATE CUT MECH. — 204

REMOVE WRAPPER MECH. — 206

REMOVE CUT LEAF — 208

FIG. 5

2/21

0004170

```
┌─────────────────────────────┐
│         INTERFACE           │──── 92
└─────────────────────────────┘
              │
             (A)
              ▼
┌─────────────────────────────┐
│           LEAF A            │
│     ACKNOWLEDGE ACCESS      │
└─────────────────────────────┘
              │
              ▼
┌─────────────────────────────┐
│       STORE CUT NO. 1       │
└─────────────────────────────┘
              │
              ▼
┌─────────────────────────────┐
│        STORE CUTTER         │
└─────────────────────────────┘
              │
              ▼
┌─────────────────────────────┐
│        STORE X1–1           │
└─────────────────────────────┘
              │
              ▼
┌─────────────────────────────┐
│        STORE X2–1           │
└─────────────────────────────┘
              │
              ▼
┌─────────────────────────────┐
│         STORE Y–1           │
└─────────────────────────────┘
              │
              ▼
┌─────────────────────────────┐
│       WAIT FOR ACCESS       │
└─────────────────────────────┘
              │
              ▼
┌─────────────────────────────┐
│      ACKNOWLEDGE ACCESS     │
└─────────────────────────────┘
              │
              ▼
┌─────────────────────────────┐
│       STORE CUT NO. 2       │
└─────────────────────────────┘
              │
              ▼
┌─────────────────────────────┐
│        STORE CUTTER         │
└─────────────────────────────┘
              │
              ▼
┌─────────────────────────────┐
│        STORE X1–2           │
└─────────────────────────────┘
              │
              ▼
┌─────────────────────────────┐
│        STORE X2–2           │
└─────────────────────────────┘
              │
              ▼
┌─────────────────────────────┐
│         STORE Y–2           │
└─────────────────────────────┘
              ┊ (N–CUTS)
              ▼
┌─────────────────────────────┐
│       ACKNOWLEDGE STOP      │
└─────────────────────────────┘
              │
              ▼
┌─────────────────────────────┐
│      INITIATE MACHINE       │
│         CONTROLLER          │
│           LEAF A            │
└─────────────────────────────┘
```

FIG. 6

FIG. 7

FIG. 7A

FIG. 7B

FIG. 10

FIG. 8

FIG. 13

FIG. 9

FIG. 14

FIG. 11

400  402  502  540  500  532  530
VIDEO  AGC E  504  AVG. VOLTAGE  ADJ. OFFSET  MODIFIED BLACK I=BK  536  536  BLACK
506  550  560  542  534  536  570  GREY  572
510  512  WHITE EXCLUDE  BLACK EXCLUDE  424  WHITE  424
514  516  ADJ.  ADJ.
520  552  562
O.I SEC  424
422  I=YES  I=YES
402  BK  W  CONVERSION
420

FIG. 12

EDGE
(HOLE) WHITE
580  OFF  AVERAGE
OFF  OFF  OFFSET
582  BLACK
VOLTAGE
X SWEEP

7/21

0004170

FIG. 15

FIG. 14A

0004170

FIG. 16

FIG. 17

FIG. 18

## FIG. 20

| MEMORY LOCATION | X TAB 662 |
|---|---|
| — 0000 | → G ADD |
| — 0001 | → W ADD |
| — 0010 | → G ADD |
| — 0011 | → W ADD |
| — 0100 | → G ADD |
| — 0101 | → B ADD |
| — 0110 | → G ADD |
| — 0111 | → W ADD |
| — 1000 | → G ADD |
| — 1001 | → W ADD |
| — 1010 | → G ADD |
| — 1011 | → B ADD |
| — 1100 | → G ADD |
| — 1101 | → B ADD |
| — 1110 | → G ADD |
| 1111 | → W ADD |

FIG. 20

## FIG. 19

| LINE | Y TAB 660 | |
|---|---|---|
| 1 | 0 | |
| 2 | 0 | |
| 3 | 2 | →G →W |
| 4 | 4 | →G →W |
| 5 | 8 | →G →B→G→W |
| 6 | 16 | →G→W→G→B→G→B→G→W |
| 7 | 22 | |
| 8 | 30 | |
| 9 | 38 | |
| 10 | 50 | |
| 11 | 58 | |
| 506 | 830 | |
| 507 | 832 | |
| 508 | 834 | |
| 509 | 834 | |
| 510 | 834 | |
| 511 | 834 | |
| 512 | 834 | |

FIG. 19

FIG. 21

VECTORIZED OUTLINE

FIG. 22

(DARK LOCATION)

FIG. 23

YI
Y2
Y3
Y4
Y5
Y6
Y7
Y8
Y9

X→B    X→G OR W

(STEM LOCATION)

X-RANGE

B    G

Y(n)
Y(n+5)
Y(n+10)
Y(n+15)
Y(n+20)
Y(n+25)
Y(n+30)

FIG. 24

Y MIN

Y
Y+1
Y+2
Y+3
Y+4
Y+5
Y+6
Y+7

Y MAX

FIG. 25

X MIN    X MAX

HOLE (WHITE DOMAIN)

PROGRAM
(OUTLINE)

(1)  HAS X TAB AND Y TAB BEEN STORED IN MEMORY (DIRECT ACCESS)?

(2)  LOOK FOR FIRST WHITE TO GRAY TRANSITION AND LAST GRAY TO WHITE TRANSITION.

(3)  STORE TRANSITIONS

(4)  REPEAT (2) AND (3) FOR EACH 40th Y LINE

(5)  CALCULATE LEAF EDGE VECTORS BETWEEN ADJACENT TRANSITIONS.

(6)  STORE LEAF EDGE VECTORS

(7)  LOOK FOR TRANSITIONS TO AND FROM WHITE IN EACH Y LINE

(8)  STORE DOMAIN OF WHITE REGION (MIN X, MIN Y; MAX X, MAX Y)

(9)  STORE HOLE DOMAINS

(10)  LOOK FOR BLACK TRANSITIONS IN EACH 5th Y LINE

(11)  CONSTRUCT STEM LINE BASED UPON REPEATED BLACK TRANSITIONS AT SAME
      POSITIONS IN ADJACENT 5th Y LINES.

(12)  CONSTRUCT SAFE LINES ON EACH SIDE OF STEM BASED UPON CONSTRUCTED
      STEM AND EXCLUDING ALL STEM BLACK

(13)  ARITHMETIC PLACEMENT OF CUT OUTLINE IN VECTOR FORM ADJACENT TO
      CONSTRUCTED SAFE LINE AT TIP OF LEAF

(14)  CHECK INTERSECTION OF LEAF EDGE VECTORS

(15)  IF NOT OK, SHIFT CUT OUTLINE ALONG SAFE LINE A GIVEN AMOUNT AND REPEAT
      UNTIL (14) CHECKS OK.

(16)  IF OK REGARDING LEAF OUTLINE, CHECK EXISTENCE OF HOLE COORDINATES WITHIN CUT
      OUTLINE. (HOLES OK IF LESS THAN SELECTED SIZE IN X AND Y DIRECTIONS)

(17)  IF NOT OK REGARDING HOLE LOCATIONS, SHIFT OUTLINE ALONG SAFE LINE A
      SELECTED DISTANCE AND RECHECK (14),(16)

(18)  IF OK REGARDING LEAF OUTLINE AND HOLE LOCATIONS, CHECK BLACK DATA AND
      EXISTENCE OF ANY HOLE IN SELECTED AREAS OF CUT PROFILE

(19)  IF NOT OK, SHIFT AND CHECK (14),(16),(18)

(20)  IF REACH OPPOSITE LEAF OUTLINE VECTOR, SHIFT Y DIRECTION CONSTRUCT NEW
      SAFE LINE AND REPEAT STEPS (13),(14),(16),(18)

(21)  REPEAT UNTIL ALL CHECKS OK OR EXHAUST LEAF HALF, WHICHEVER COMES FIRST

(22)  WHEN ALL OK, NOTE X1, X2 AND Y COORDINATES FOR SELECTED CUT OUTLINE POSITION

(23)  STORE CUT COORDINATES

(24)  ADD CUT OUTLINE VECTORS AT X1, X2 AND Y COORDINATES TO LEAF OUTLINE VECTORS

(25)  REPEAT FOR NEXT CUT FROM (13) OR CUTS ON SECOND LEAF HALF

(26)  REPEAT FOR SECOND LEAF HALF FROM STEP (12)

(27)  CREATE READY SIGNAL

(28)  DOES PROGRAMMABLE CONTROLLER WANT DATA?

(29)  IF NO, WAIT FOR YES

(30)  IF YES, TRANSFER DATA TO PROGRAMMABLE CONTROLLER

FIG. 26

FIG. 27

FIG. 28

FIG. 29

FIG. 30

FIG. 31

FIG. 32

FIG. 33

FIG. 34

FIG. 35

FIG. 36

FIG. 36A

FIG. 36B

FIG. 37

960-1 960-2 900 960-3 960-4 960-5 960-6 960-7 960-8
962-1 962-2 962-3 962-4 962-5 962-6 38 962-7 962-8
750 754a 930 950 930a 952 910
966 964
963 974 972 974 972 974 972 974
974 972 974 972 974 972 VALVES
970
8 BIT

FIG. 38

990 900
988
966
989 992
963

(.040) (.080) (.160) (.320) (.640) (1.28) (2.56) (5.12)
ON OFF 974
972
965-1 965-2 965-3 965-4 965-5 965-6 965-7 965-8
BIT 1 BIT 2 BIT 3 BIT 4 BIT 5 BIT 6 BIT 7 BIT 8

I = ON
O = OFF    EXAMPLE: 01001000 = .080 + .640 = .720

FIG. 39

CUT → X1 (8 BITS)
→ X2 (8 BITS)
→ Y (8 BITS)

FIG. 40

FIG. 40A

FIG. 41

1230
1220
1210
SCAN
X Y
SCAN

1226 L
1216
1240 (AIR JET)
LOAD

1200
1212 1222 1234
CUT
TRANSFER

1214
1224
UNLOAD

FIG. 42

GRID A 1300
SCAN

TRANSFER
GRID B 1302
SHIFT
1304
CUT 1302

1232 1230
1232
L
1220
VAC

FIG. 43

European Patent Office

**EUROPEAN SEARCH REPORT**

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| | DE - A - 2 538 964 (HANNI-WERKE KOERBER)<br>  * Figures 1-2; pages 6-11 *<br>-- | 1 | A 24 C  1/04<br>G 06 F  15/20<br>B 26 D  5/34 |
| | GB - A - 1 228 637 (COMPAGNIE DE SAINT-GOBAIN)<br>  * Figures 4,5; page 4, line 11 - page 5, line 101 *<br>-- | 1 | |
| | FR - A - 1 580 133 (GLAVERBEL)<br>  * Page 6, left-hand column, line 18 - page 7, left-hand column, line 22 *<br>-- | 1 | TECHNICAL FIELDS SEARCHED (Int.Cl.²)<br><br>A 24 C<br>B 07 C<br>G 06 F<br>B 26 D<br>C 03 B |
| | FR - A - 2 107 099 (AMP INC.)<br>  * Figure 6; page 9, lines 8-32 *<br>-- | 3-6,8 | |
| P | FR - A - 2 373 240 (ARENCO)<br>  * Figures; pages 2-3 *<br>-- | 1,8-10 13-15, 29-34 | |
| P | NL - A - 78 08627 (DOUWE EGBERTS)<br>  * Page 2, line 9 - page 3, line 11; claims 1-6 *<br>& FR - A - 2 400 852<br>& GB - A - 2 004 643<br>---- | 1,29-34 | CATEGORY OF CITED DOCUMENTS<br><br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br><br>&: member of the same patent family, corresponding document |

The present search report has been drawn up for all claims

| Place of search | Date of completion of the search | Examiner |
|---|---|---|
| The Hague | 15-06-1979 | RIEGEL |

EPO Form 1503.1  06.78